# EUROPÄISCHE PATENTSCHRIFT

(11) **EP 1 060 162 B1**
(45) Veröffentlichungstag und Bekanntmachung des Hinweises auf die Patenterteilung: **19.03.2003**
(21) Anmeldenummer: 98962423.4
(22) Anmeldetag: 08.12.1998
(51) Int. Cl.: C07D 211/54, C07D 211/32, C07D 211/26, C07D 211/70, A61K 31/445

(54) **VON AZACYCLOALKANEN ABGELEITETE URETHANE, IHRE THIO- UND DITHIOANALOGA, DEREN HERSTELLUNG UND DEREN VERWENDUNG ALS 2,3-EPOXISQUALEN-LANOSTEROL-CYCLASE INHIBITOREN**
URETHANES DERIVED FROM AZACYCLOALKANES, THIO AND DITHIO ANALOGUES, PRODUCTION AND USE THEREOF AS 2,3 EPOXYSQUALENE LANESTEROL CYCLASE INHIBITORS
URETHANES DERIVES D'AZACYCLOALCANES, LEURS ANALOGUES THIO ET DITHIO, LEUR PRODUCTION ET LEUR UTILISATION COMME INHIBITEURS DE LA 2,3-EPOXYSQUALENE-LANOSTEROL-CYCLASE

(30) Priorität: 10.12.1997 DE 19754796
(43) Veröffentlichungstag der Anmeldung: 20.12.2000
(73) Patentinhaber: Boehringer Ingelheim Pharma KG, 55218 Ingelheim am Rhein (DE)
(72) Erfinder: MAIER, Roland, D-88400 Biberach (DE); MÜLLER, Peter, Stamford, CT 06903 (US); SCHILCHER, Gebhard, D-88441 Mittelbiberach (DE); ADELGOSS, Gebhard, D-88400 Biberach (DE); HURNAUS, Rudolf, D-88400 Biberach (DE); MARK, Michael, D-88400 Biberach (DE); EISELE, Bernhard, D-88400 Biberach (DE)
(74) Vertreter: Laudien, Dieter, Dr.
(86) Internationale Anmeldenummer: EP9807965
(87) Internationale Veröffentlichungsnummer: WO99029669

(56) Entgegenhaltungen:
- DE-A- 4 407 136
- DE-A- 4 407 138
- CHEMICAL ABSTRACTS, vol. 119, no. 5, 2. August 1993 Columbus, Ohio, US; abstract no. 49233t, GOTO G. ET AL.: "Preparation of 4-benzylpiperidine derivatives for treating edema of brain" Seite 929; Spalte 1; XP002100900 & JP 04 312572 A (TAKEDA CHEMICAL INDUSTRIES, LTD.) 12. April 1991
- CHANG G. & RUGGERI R.B.: "Recent developments in the treatment of dyslipidaemia" EXPERT OPINION ON THERAPEUTIC PATENTS, Bd. 7, Nr. 5, Mai 1997, Seiten 441-455, XP002100899 in der Anmeldung erwähnt

## Beschreibung

Die vorliegende Erfindung betrifft neue, von Azacycloalkanen abgeleitete Urethane, ihre Thio- und Dithioanaloga, deren Salze mit physiologisch verträglichen organischen und anorganischen Säuren, Verfahren zur Herstellung dieser Verbindungen und diese enthaltende Arzneimittel.

Die erfindungsgemäßen Verbindungen stellen Inhibitoren der Cholesterolbiosynthese dar, insbesondere Inhibitoren des Enzyms 2,3-Epoxisqualen-Lanosterol-Cyclase, eines Schlüsselenzyms der Cholesterolbiosynthese. Die erfindungsgemäßen Verbindungen sind geeignet zur Behandlung und Prophylaxe von Hyperlipidämien, Hypercholesterolämien und der Atherosklerose. Weitere mögliche Anwendungsgebiete ergeben sich für die Behandlung von hyperproliferativen Haut- und Gefässerkrankungen, Tumoren, Gallensteinleiden sowie von Mykosen.

Verbindungen,die in die Cholesterolbiosynthese eingreifen, sind für die Behandlung einer Reihe von Krankheitsbildern von Bedeutung. Hier sind vor allem Hypercholesterolämien und Hyperlipidämien zu nennen, die Risikofaktoren für das Entstehen atherosklerotischer Gefäßveränderungen und ihrer Folgeerkrankungen wie beispielsweise koronare Herzkrankheit, cerebrale Ischämie, Claudicatio intermittens und Gangrän darstellen.

Die Bedeutung überhöhter Serum-Cholesterol-Spiegel als Hauptrisikofaktor für das Entstehen atherosklerotischer Gefäßveränderungen wird allgemein anerkannt. Umfangreiche klinische Studien haben zu der Erkenntnis geführt, daß durch Erniedrigung des Serumcholesterols das Risiko, an koronaren Herzkrankheiten zu erkranken, verkleinert werden kann (Current Opinion in Lipidology 2(4), 234 [1991]; Exp. Opin. Ther. Patents 7(5), 441-455 [1997]). Da der größte Teil des Cholesterols im Organismus selbst synthetisiert und nur ein geringer Teil mit der Nahrung aufgenommen wird, stellt die Hemmung der Biosynthese einen besonders attraktiven Weg dar, den erhöhten Cholesterolspiegel zu senken.

Daneben werden als weitere mögliche Anwendungsgebiete von Cholesterolbiosynthesehemmern die Behandlung hyperproliferativer Haut-und Gefäßerkrankungen sowie von Tumorerkrankungen, die Behandlung und Prophylaxe von Gallensteinleiden sowie der Einsatz bei Mykosen beschrieben. Hierbei handelt es sich im letzten Fall um einen Eingriff in die Ergosterolbiosynthese in Pilzorganismen, welche weitgehend analog der Cholesterolbiosynthese in Säugerzellen verläuft.

Die Cholesterol- bzw. die Ergosterolbiosynthese verläuft, ausgehend von Essigsäure, über eine größere Zahl von Reaktionsschritten. Dieser Vielstufenprozeß bietet eine Reihe von Eingriffsmöglichten, von denen als Beispiele genannt seien:

Für die Inhibition des Enzyms 3-Hydroxy-3-methylglutaryl-coenzym A (HMG-CoA)-Synthase werden β-Lactone-und β-Lactame mit potentieller antihypercholesterolämischer Wirkung erwähnt (siehe J. Antibiotics 40, 1356 [1987], US-A-4,751,237, EP-A-0 462 667, US-A-4,983,597).

Beispiele für Inhibitoren des Enzyms HMG-CoA-Reduktase stellen 3,5-Dihydroxycarbonsäuren vom Mevinolintyp und deren δ-Lactone dar, deren Vertreter Lovastatin, Simvastatin, Pravastatin, Fluvastatin, Atorvastatin sowie Cerivastatin in der Therapie von Hypercholesterolämien Verwendung finden. Weitere mögliche Anwendungsgebiete dieser Verbindungen sind Pilzinfektionen (US-A-4,375,475, EP-A-0 113 881, US-A-5,106,592), Hauterkrankungen (EP-A-0 369 263) sowie Gallensteinleiden und Tumorerkrankungen (US-A-5,106,992; Lancet 339, 1154-1156 [1992]). Die Hemmung der Proliferation glatter Muskelzellen durch Lovastatin ist beschrieben in Cardiovasc. Drugs. Ther. 5, Suppl. 3, 354 [1991]. Tocotrienol, ein ungesättigtes Analoges des Vitamin E, und dessen Analoga stellen eine weitere Substanzklasse dar, die auf die HMG-CoA-Reduktase einwirkt (Exp. Opin. Ther. Patents 7 (5), 446 [1997]).

Inhibitoren des Enzyms Squalen-Synthetase sind z.B. Isoprenoid -(phosphinylmethyl)-phosphonate, deren Eignung zur Behandlung von Hypercholesterolämien, Gallensteinleiden und Tumorerkrankungen in EP-A-0 409 181 sowie in J. Med. Chemistry 34, 1912 [1991] beschrieben ist, ferner α-Phosphonosulfinat-Verbindungen (EP-A-0 698 609), die Verbindungen J-104,118 und J-104,123 (Tetrahedron 52, 13881-13894, [1996]) sowie Cyclobutanderivate (WO 96/33159). Ein Überblick über Squalen-Synthethase-Inhibitoren findet sich in Exp. Opin. Ther. Patents 7 (5), 446-448 [1997].

Als Inhibitoren des Enzyms Squalen-Epoxidase sind bekannt Allylamine wie Naftidin und Terbinafin, die als Mittel gegen Pilzerkrankungen Eingang in die Therapie gefunden haben, sowie das Allylamin NB-598 mit antihypercholesterolämischer Wirkung (J. Biol. Chemistry 265, 18075-18078 [1990]) und Fluorsqualen-Derivate mit hypocholesterolämischer Wirkung (US-A-5,011,859). Des weiteren sind Piperidine und Azadecaline mit potentieller hypocholesterolämischer und/oder antifungaler Wirkung beschrieben, deren Wirkmechanismus nicht eindeutig geklärt ist und welche Squalenepoxidase- und/oder 2,3-Epoxisqualen-Lanosterol-Cyclase-Inhibitoren darstellen (EP-A-0 420 116, EP-A-0 468 434, US-A-5,084,461 und EP-A-0 468 457). Weitere Vertreter sind beschrieben in Exp. Opin. Ther. Patents 7 (5), 448-449 [1997].

Beispiele für Inhibitoren des Enzyms 2,3-Epoxisqualen-Lanosterol-Cyclase sind Diphenylderivative (EP-A-0 464 465), Aminoalkoxybenzol-Derivate (EP-A-0 410 359, J. Lipid. Res. 38, 373-390, [1997]) sowie Piperidin-Derivate (J. Org. Chem. 57, 2794-2903 [1992], die eine antifungale Wirkung besitzen. Des weiteren wird dieses Enzym in Säugetierzellen durch Decaline, Azadecaline und Indanderivate (WO 89/08450; J. Biol. Chemistry 254, 11258-11263 [1981]; Biochem. Pharmacology 37, 1955-1964 [1988] und J 64 003 144), ferner durch 2-Aza-2,3-dihydrosqualen und 2,3-Epiminosqualen (Biochem. Pharmacology 34, 2765-2777 [1985]), durch Squalenoid-Epoxid-Vinylether (J. Chem. Soc. Perkin Trans. I, 461 [1988]) und 29-Methyliden-2,3-oxidosqualen (J. Amer. Chem. Soc. 113, 9673-9674 [1991]) inhibiert. Weitere Beispiele sind Pyridin-bzw. Pyrimidin-Derivate (WO 97/06802), heterobicyclische Alkylamine (WO 96/11201), Imidazolderivate (EP-A-0 757 988) sowie Isochinolinderivate (J. Med. Chemistry 39, 2302-2312, [1996]). Des weiteren sind beschrieben Harnstoffe (DE-A-4 438 021), Oxime (DE-A-4 412 692), eine Reihe von Amiden (DE-A-4 407 134, DE-A-4 407 135, DE-A-4 407 136, DE-A-4 407 138, DE-A-4 407 139, DE-A-4 412 691, DE-A-4 437 999, DE-A-4 438 000, DE-A-4 438 020, DE-A-4 438 082, DE-A-4 438 029, DE-A-4 438 054, DE-A-4 438 055, DE-A-4 438 082, DE-A-4 438 083, EP-A-0 599 203, EP-A-0 596 326) sowie Ester (WO 95/29148). Weitere Beispiele sind beschrieben in Exp. Opin. Ther. Patents 7(5), 448-449 [1997].

Schließlich sind als Inhibitoren des Enzyms Lanosterol-14α-Demethylase noch Steroidderivate mit potentieller antihyperlipidämischer Wirkung zu nennen, die gleichzeitig das Enzym HMG-CoA-Reduktase beeinflussen (US-A-5,041,431; J.Biol. Chemistry 266, 20070-20078 [1991]; US-A-5,034,548). Außerdem wird dieses Enzym durch die Antimykotika vom Azol-Typ inhibiert, welche N-substituierte Imidazole und Triazole darstellen. Zu dieser Klasse gehören beispielsweise die auf dem Markt befindlichen Antimykotika Ketoconazol und Fluconazol.

Die Verbindungen der nachfolgenden allgemeinen Formel I sind neu. Es wurde überraschenderweise gefunden, daß sie sehr wirksame Inhibitoren des Enzyms 2,3-Epoxisqualen-Lanosterol-Cyclase (Internationale Klassifizierung: EC 5.4.99.7) darstellen.

Das Enzym 2,3-Epoxisqualen-Lanosterol-Cyclase katalysiert einen Schlüsselschritt der Cholesterol- bzw. Ergosterol-Biosynthese, nämlich die Umwandlung des 2,3-Epoxisqualens in das Lanosterol, die erste Verbindung mit Steroidstruktur in der Biosynthesekaskade. Inhibitoren dieses Enzyms lassen gegenüber Inhibitoren früherer Biosyntheseschritte, wie beispielsweise HMG-CoA-Synthase und HMG-CoA-Reduktase, den Vorteil der höheren Selektivität erwarten, da die Inhibierung dieser frühen Biosyntheseschritte zur Abnahme biosynthetisch gebildeter Mevalonsäure führt und dadurch auch die Biosynthese der mevalonsäureabhängigen Substanzen Dolichol, Ubichinon und Isopentenyl-t-RNA negativ beeinflussen kann (vgl. J. Biol. Chemistry 265, 18075-18078 [1990].

Bei Inhibierung von Biosyntheseschritten nach der Umwandlung von 2,3-Epoxisqualen in Lanosterol besteht die Gefahr der Anhäufung von Intermediärprodukten mit Steroidstruktur im Organismus und der Auslösung dadurch bedingter toxischer Effekte. Dies ist beispielsweise für Triparanol, einem Desmosterol-Reduktase-Inhibitor, beschrieben. Diese Substanz mußte wegen Bildung von Katarakten, Ichthyosis und Alopecie vom Markt genommen werden (zitiert in J. Biol. Chemistry 265, 18075-18078 [1990]).

Wie bereits eingangs dargelegt sind Inhibitoren der 2,3-Epoxisqualen-Lanosterol-Cyclase bereits in der Literatur beschrieben. Es sind jedoch keinerlei Urethane sowie deren Thiooder Dithioanaloge als Inhibitoren der 2,3-Epoxisqualen-Lanosterol-Cyclase bekannt.

Die Erfindung betrifft die Bereitstellung von antihypercholesterolämischen Substanzen, die zur Behandlung und Prophylaxe der Atherosklerose geeignet sind und im Vergleich zu bekannten Wirkstoffen durch eine bessere antihypercholesterolämische Wirkung bei erhöhter Selektivität und damit erhöhter Sicherheit ausgezeichnet sind. Da die erfindungsgemäßen Verbindungen auf Grund ihrer hohen Wirksamkeit als Inhibitoren des Enzyms 2,3-Epoxisqualen-Lanosterol-Cyclase auch die Ergosterol-Biosynthese im Pilzorganismus inhibieren können, sind sie auch zur Behandlung von Mykosen geeignet.

Die vorliegende Erfindung betrifft die neuen, von Azacycloalkanen abgeleiteten Urethane sowie deren Thio- und Dithioanaloga der allgemeinen Formel 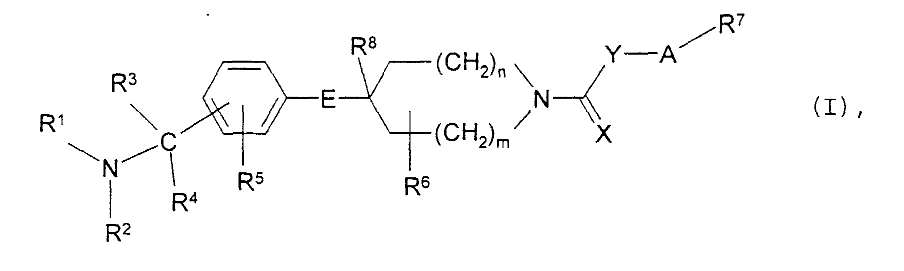 in der
m die Zahlen 0 oder 1,
n die Zahlen 1 oder 2,
A eine Einfachbindung, eine geradkettige oder verzweigte C₁₋₈-Alkylengruppe, eine C₂₋₈-Alkenylen- oder C₂₋₈-Alkinylengruppe, wobei eine ungesättigte Gruppe nicht direkt an den Rest Y gebunden ist,
X ein Sauerstoff- oder Schwefelatom,
Y ein Sauerstoff- oder Schwefelatom,
R¹ eine geradkettige oder verzweigte C₁₋₆-Alkylgruppe, eine C₁₋₄-Alkenylgruppe oder eine C₁₋₄-Alkinylgruppe, wobei die Mehrfachbindung von der Stickstoff-Kohlenstoff-Bindung isoliert ist,
R² eine geradkettige oder verzweigte C₁₋₆-Alkylgruppe, eine C₁₋₄-Alkenyl- oder C₁₋₄-Alkinylgruppe, wobei eine Mehrfachbindung von der Stickstoff-Kohlenstoff-Bindung isoliert ist, oder
R¹ und R² zusammen mit dem Stickstoffatom einen 5-bis 7-gliedrigen, gesättigten heterocyclischen Ring, in dem eine von dem Stickstoffatom isolierte Methylengruppe durch ein Sauerstoffoder Schwefelatom oder durch eine -NH- oder -N(Alkyl)-Gruppe ersetzt sein kann,
R³ bis R⁶, die gleich oder verschieden sein können, Wasserstoffatome oder Alkylgruppen,
R⁷ eine C₃₋₇-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe oder, sofern A keine Einfachbindung darstellt, auch ein Wasserstoffatom,
E ein Sauerstoff- oder Schwefelatom, eine Methylen-, Carbonyloder Sulfinylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten, wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
deren Enantiomere, Diastereomere, deren Gemische und deren Salze, insbesondere deren physiologisch verträglichen Säureadditionssalze.

Bevorzugt sind Verbindungen der allgemeinen Formel I, in der
m die Zahl 1,
n die Zahl 1,
A eine Einfachbindung, eine geradkettige oder verzweigte C₁₋₄-Alkylengruppe oder eine C₂₋₄-Alkenylengruppe, wobei eine ungesättigte Gruppe nicht direkt an den Rest Y gebunden ist,
X ein Sauerstoff- oder Schwefelatom,
Y ein Sauerstoff- oder Schwefelatom,
R¹ eine geradkettige oder verzweigte C₁₋₆-Alkylgruppe, eine Allyl- oder Propargylgruppe, wobei die Mehrfachbindung von der Stickstoff-Kohlenstoff-Bindung isoliert ist,
R² eine geradkettige oder verzweigte C₁₋₆-Alkylgruppe, eine Allyl- oder Propargylgruppe, wobei die Mehrfachbindung von der Stickstoff-Kohlenstoff-Bindung isoliert ist, oder
R¹ und R² zusammen mit dem Stickstoffatom einen 5- bis 7-gliedrigen, gesättigten heterocyclischen Ring, in dem eine von dem Stickstoffatom isolierte Methylengruppe durch ein Sauerstoffoder Schwefelatom ersetzt sein kann,
R³ bis R⁶, die gleich oder verschieden sein können, Wasserstoffatome oder Methylgruppen,
R⁷ eine C₃₋₆-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe,
E ein Schwefelatom, eine Methylen-, Carbonyl- oder Sulfinylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten, wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
deren Enantiomere, Diastereomere, deren Gemische und deren Salze, insbesondere deren physiologisch verträglichen Säureadditionssalze.
Besonders bevorzugt sind die Verbindungen der allgemeinen Formel I, in der
m die Zahl 1,
n die Zahl 1,
A eine Einfachbindung oder eine geradkettige oder verzweigte C₁₋₃-Alkylengruppe,
X ein Sauerstoff- oder Schwefelatom,
Y ein Sauerstoff- oder Schwefelatom,
R¹ eine geradkettige oder verzweigte C₁₋₃-Alkylgruppe,
R² eine geradkettige oder verzweigte C₁₋₃-Alkylgruppe oder
R¹ und R² zusammen mit dem Stickstoffatom eine Piperidino- oder Morpholinogruppe,
R³ bis R⁶ Wasserstoffatome,
R⁷ eine Cyclohexylgruppe oder eine gegebenenfalls durch ein Halogenatom, durch eine Alkyl-, Alkoxy- oder Trifluormethylgruppe substituierte Phenylgruppe,
E ein Schwefelatom, eine Methylengruppe, eine Carbonyl- oder Sulfinylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten,
deren Enantiomere, Diastereomere, deren Gemische und deren Salze, insbesondere deren physiologisch verträglichen Säureadditionssalze.

Ganz besonders bevorzugt sind die Verbindungen der allgemeinen Formel I, in der
m die Zahl 1,
n die Zahl 1,
A eine Einfachbindung oder eine Methylengruppe,
X ein Sauerstoff- oder Schwefelatom,
Y ein Sauerstoff- oder Schwefelatom,
R¹ und R² jeweils eine Methylgruppe,
R³ bis R⁶ Wasserstoffatome,
R⁷ eine gegebenenfalls durch ein Fluor- oder Chloratom oder durch eine Methylgruppe substituierte Phenylgruppe,
E ein Schwefelatom, eine Methylen- oder Carbonylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten, deren Gemische und deren Salze, insbesondere deren physiologisch verträglichen Säureadditionssalze,
insbesondere jedoch die Verbindungen
(1) N-(Benzylthio)thiocarbonyl-4-[4-(dimethylaminomethyl)-phenylthio]piperidin,
(2) N-(Benzylthio)thiocarbonyl-4-[4-(dimethylaminomethyl)benzoyl]piperidin,
(3) N-Benzyloxycarbonyl-4-[4-(dimethylaminomethyl)phenylthio]-piperidin,
(4) N-(4-Chlorphenoxy)carbonyl-4-[4-(dimethylaminomethyl)phenylthio] piperidin,
(5) N-(Benzylthio)thiocarbonyl-4-[4-(dimethylaminomethyl)benzyl]piperidin,
(6) N-(Benzylthio)thiocarbonyl-4-[4-(dimethylaminomethyl)benzyliden]piperidin,
(7) N-(4-Chlorphenoxy)thiocarbonyl-4-[4-(dimethylaminomethyl)-benzyl]piperidin,
(8) N-(4-Chlorphenoxy)carbonyl-4-[4-(dimethylaminomethyl)-benzyl]piperidin,
(9) N-Benzyloxycarbonyl-4-[4-(dimethylaminomethyl)benzyl]-piperidin,
(10) 4-[4-(Dimethylaminomethyl)benzyl]-N-(4-methylphenoxy)-carbonylpiperidin,
(11) 4-[4-(Dimethylaminomethyl)benzyl]-N-(4-methylphenoxy)-thiocarbonylpiperidin und
(12) 4- [4-(Dimethylaminomethyl)benzyl]-N-(4-fluorphenoxy)-carbonylpiperidin,
deren Gemische und deren Salze, insbesondere deren physiologisch verträglichen Säureadditionssalze, beispielsweise deren Hydrochloride, Methansulfonate und Tartrate.

Die Verbindungen der allgemeinen Formel I lassen sich beispielsweise nach folgenden Methoden herstellen:
a) Umsetzung einer Verbindung der allgemeinen Formel 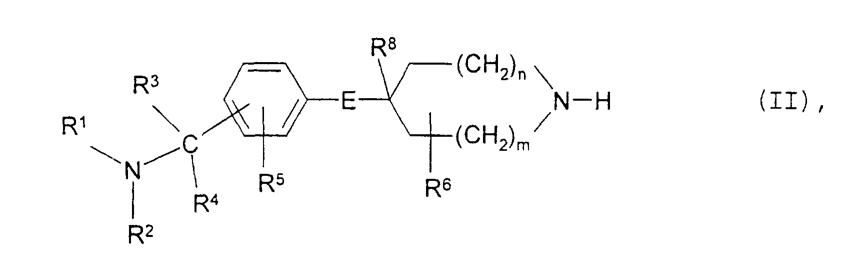 in der
   m, n, E mit Ausnahme der Sulfinylgruppe, R¹ bis R⁶ und R⁸ wie eingangs erwähnt definiert sind, mit einer Verbindung der allgemeinen Formel 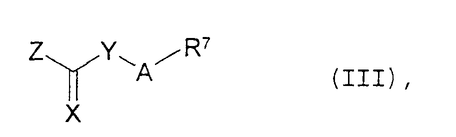 in der
   A, X, Y und R⁷ wie eingangs erwähnt definiert sind und Z eine Austrittsgruppe, beispielsweise ein Halogenatom, wie das Chlor-, Brom- oder Iodatom, bedeutet,
   Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, N-Ethyl-diisopropylamin, N-Ethyl-dicyclohexylamin, 1,4-Diazabicyclo[2,2,2]octan oder 1,8-Diazabicyclo[5,4,0]undec-7-en, als Lösemittel beispielsweise Diethylether, Methylenchlorid, Dichlormethan, Ethylacetat, Toluol, Tetrahydrofuran, 1,4-Dioxan, Acetonitril, Dimethylformamid, Dimethylacetamid, N-Methyl-pyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.
b) Zur Herstellung von Verbindungen der allgemeinen Formel (I), in der X und Y jeweils ein Schwefelatom bedeuten und m, n, A, E und R¹ bis R⁸ mit der Maßgabe wie eingangs erwähnt definiert sind, daß R⁷ keine gegebenenfalls substituierte Phenyloder Naphtylgruppe darstellt, falls A eine Einfachbindung bedeutet:
   Umsetzung von Verbindungen der allgemeinen Formel (II), in der m ,n ,E mit Ausnahme der Sulfinylgruppe, R¹ bis R⁶ und R⁸ wie eingangs erwähnt definiert sind, mit Schwefelkohlenstoff und anschließend mit einem Alkylierungsmittel der allgemeinen Formel 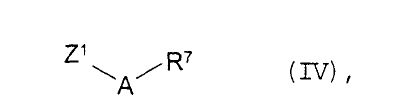 in der
   A und R⁷ mit der Maßgabe wie eingangs erwähnt definiert sind, daß R⁷ keine gegebenenfalls substituierte Phenyl- oder Naphtylgruppe darstellt, falls A eine Einfachbindung bedeutet, und Z¹ eine Austrittsgruppe, beispielsweise ein Halogenatom, wie das Chlor-, Brom- oder Iodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxyoder Naphthylsulfonyloxygruppe, wobei die Substituenten gleich oder verschieden sein können, bedeutet,
   Die Umsetzung wird zweckmäßigerweise so durchgeführt, daß eine Verbindung der allgemeinen Formel (II) in einem geeigneten Lösungsmittel, beispielsweise in Tetrahydrofuran, Dioxan, Hexan oder Toluol, zunächst in das Lithiumsalz überführt wird, beispielsweise mit n-Butyllithium bei einer Temperatur von -20 bis -10°C, und dann mit Schwefelkohlenstoff umgesetzt wird. Anschließend wird eine Verbindung der allgemeinen Formel (IV) in einem geeigneten Lösungsmittel, beispielsweise in Tetrahydrofuran, Dimethylformamid, Dimethylsulfoxid oder einem Gemisch davon, zugegeben und die Umsetzung bei 20-60°C durchgeführt.
c) Zur Herstellung von Verbindungen der allgemeinen Formel (I), in der E eine Sulfinylgruppe bedeutet:
   Oxidation einer Verbindung der allgemeinen Formel (I), in der m, n, A, X, Y, und R¹ bis R⁸ wie eingangs erwähnt definiert sind und E ein Schwefelatom bedeutet, vorzugsweise mit Natrium-meta-perjodat.
   Die Oxidation wird zweckmäßigerweise mit einem Äquivalent des verwendeten Oxidationsmittels durchgeführt, z.B. mit Natriummeta-perjodat in wäßrigem Methanol oder Ethanol bei -15 bis 25°C.

Die nach den vorstehenden Verfahren hergestellten Verbindungen der allgemeinen Formel I lassen sich nach bekannten Methoden reinigen und isolieren, beispielsweise mittels Kristallisation, Destillation oder Chromatographie.

Ferner können die erhaltenen Verbindungen der allgemeinen Formel I gegebenenfalls in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschlie8end wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Die Ausgangsverbindungen der allgemeinen Formel II, in der E eine Carbonylgruppe bedeutet, lassen sich nach den in DE 44 07 136 A1 (S. 4-5) beschriebenen Methoden herstellen.

Die Ausgangsverbindungen der allgemeinen Formel II, in der E eine Methylengruppe oder die Gruppe -C(R⁹R¹⁰)- bedeutet, wobei R⁹ ein Wasserstoffatom und R¹⁰ zusammen mit der benachbarten Gruppe R⁸ eine Kohlenstoff-Kohlenstoff-Bindung darstellt, lassen sich nach den in DE 44 07 138 A1 (5/1144) (S. 7-8) beschriebenen Methoden herstellen.

Verbindungen der allgemeinen Formel II, in der E ein Sauerstoff- oder Schwefelatom bedeutet, lassen sich nach folgendem Reaktionsschema herstellen:

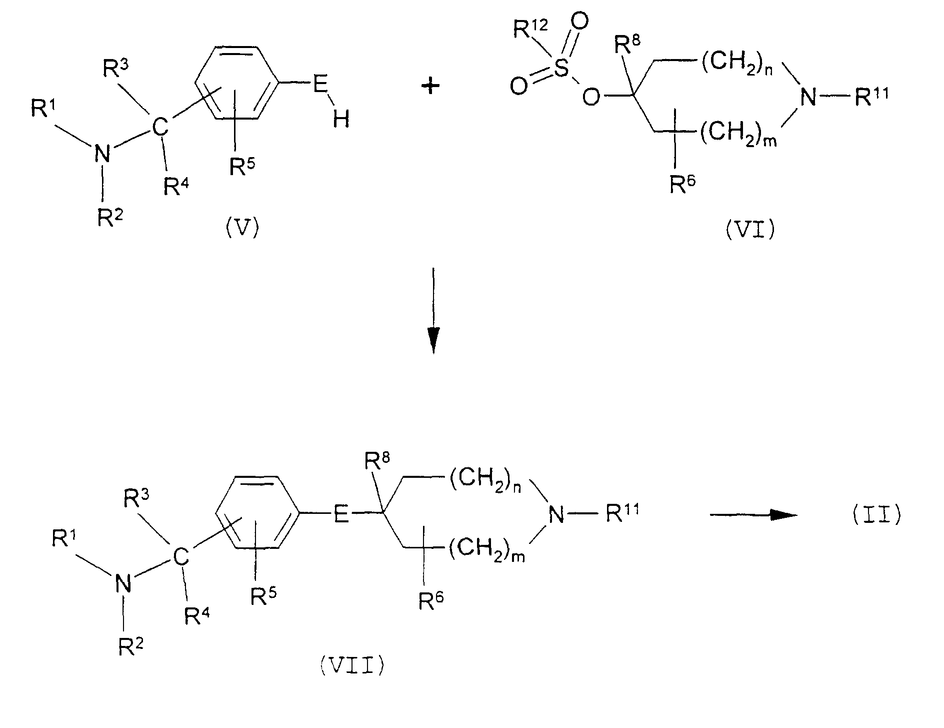

Die Umsetzung einer Verbindung der Formel V, in der R¹ bis R⁵ wie eingangs erwähnt definiert sind und E ein Sauerstoff- oder Schwefelatom bedeutet, mit einer Verbindung der Formel VI, in der m, n, R⁶ und R⁸ wie eingangs erwähnt definiert sind, R¹¹ eine Schutzgruppe, beispielsweise die tert.-Butyloxycarbonylgruppe, und R¹² einen Alkylrest bedeutet, liefert eine entsprechende Verbindung der Formel VII, die durch Abspaltung des Schutzrestes R¹¹ in eine Verbindung der Formel II umgewandelt wird.

Verbindungen der allgemeinen Formel II, in der E eine Methylengruppe, ein Sauerstoff- oder Schwefelatom, R³ bis R⁵ und R⁸ Wasserstoffatome, R⁶ ein Wasserstoffatom oder eine Alkylgruppe bedeuten und der Phenylrest 1,4-disubstituiert ist, lassen sich nach den beiden, im folgendem Reaktionsschema dargestellten Methoden herstellen:

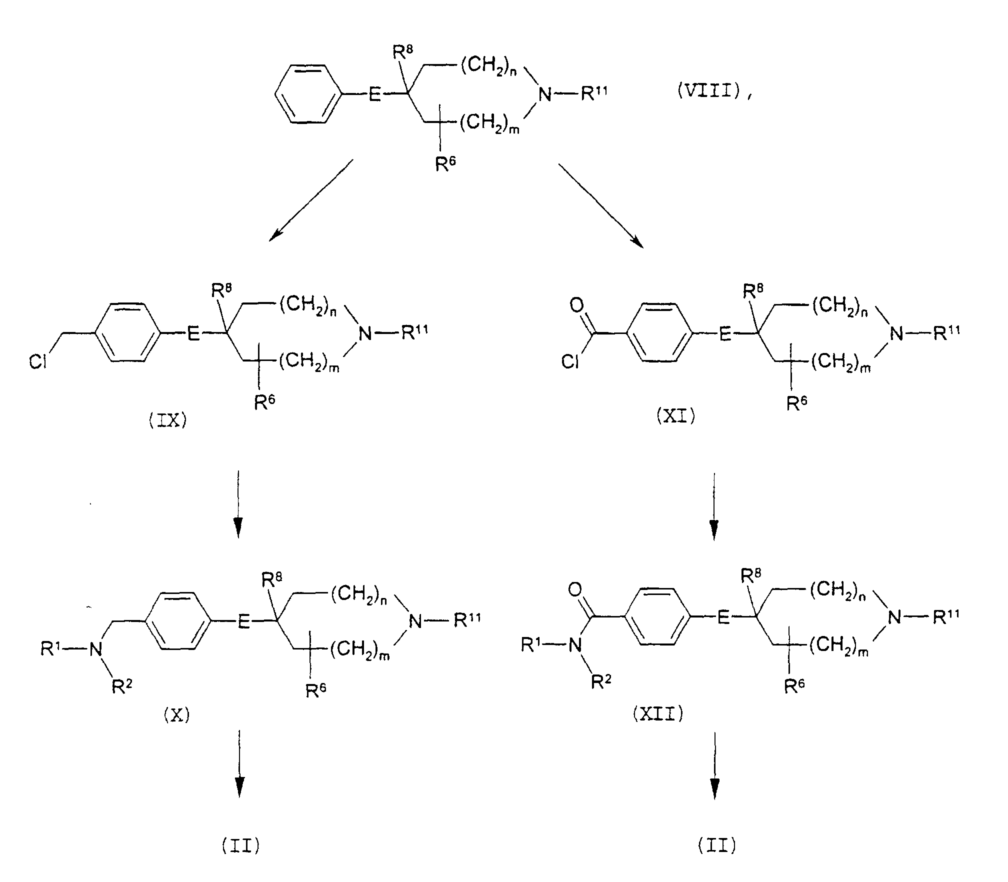

Einerseits wird eine Verbindung der Formel VIII, in der R¹¹ eine Schutzgruppe darstellt, vorzugsweise eine 2.2.2-Trichlorethoxycarbonylgruppe, durch Chlormethylierung in eine Verbindung der Formel IX überführt. Aminolyse mit einem Amin der Formel R¹R²NH ergibt eine Verbindung der Formel X, die durch Abspaltung der Schutzgruppe in eine Verbindung der Formel II umgewandelt wird.

Andererseits kann eine Verbindung der Formel VIII, in der R¹¹ eine Schutzgruppe darstellt, vorzugsweise den Trifluoracetylrest, durch eine Friedel-Crafts-Reaktion mit Oxalylchlorid in Gegenwart von Aluminiumchlorid in eine Verbindung der Formel XI umgewandelt werden. Aminolyse mit einem Amin der Formel R¹R²NH ergibt eine Verbindung der Formel XII, die durch Reduktion mit Lithiumaluminiumhydrid und Abspaltung des Schutzrestes in eine Verbindung der Formel II überführt wird.

Verbindungen der Formel II, in der E ein Sauerstoff- oder Schwefelatom, R³ bis R⁵ und R⁸ Wasserstoffatome, R⁶ ein Wasserstoffatom oder eine Alkylgruppe bedeuten, lassen sich auch nach folgender Methode darstellen:

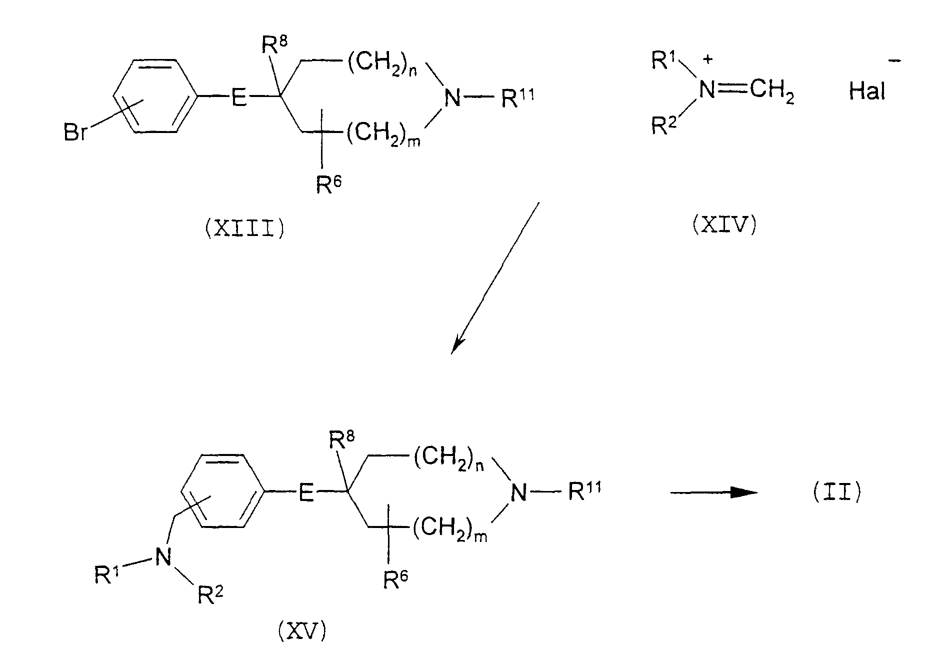

Eine Verbindung der Formel XIII, in der R¹¹ eine Schutzgruppe darstellt, vorzugsweise einen Tritylrest, wird zunächst in die Lithiumverbindung überführt und dann mit einer Verbindung der Formel XIV, in der Hal ein Chlor-, Brom- oder Jodatom bedeutet, umgesetzt zu einer Verbindung der Formel XV. Abspaltung der Schutzgruppe gibt eine Verbindung der Formel II.

Die Verbindungen der allgemeinen Formel I besitzen interessante biologische Eigenschaften. Sie stellen Inhibitoren der Cholesterolbiosynthese, insbesondere Inhibitoren des Enzyms 2,3-Epoxisquaien-Lanosterol-Cyclase dar. Aufgrund ihrer biologischen Eigenschaften sind sie geeignet zur Behandlung von Krankheiten, bei denen die Cholesterol-Biosynthese eine Rolle spielt, insbesondere zur Behandlung und Prophylaxe der Hypercholesterolämie, der Hyperlipoproteinämie und der Hypertriglyceridämie und den daraus resultierenden atherosklerotischen Gefäßveränderungen mit ihren Folgeerkrankungen wie koronare Herzkrankheit, cerebrale Ischämie, Claudicatio intermittens, Gangrän und andere.

Zur Behandlung dieser Erkrankungen können die Verbindungen der allgemeinen Formel I dabei entweder alleine zur Monotherapie eingesetzt werden oder in Kombination mit anderen cholesteroloder lipidsenkenden Substanzen zur Anwendung gelangen, wobei die Verbindungen vorzugsweise als orale Formulierung, gegebenenfalls auch in Form von Suppositorien als rektale Formulierung verabreicht werden können. Als Kombinationspartner kommen dabei beispielsweise in Frage:
- gallensäurebindende Harze wie z. B. Cholestyramin, Cholestipol und andere,
- Verbindungen, die die Cholesterolresorption hemmen, wie z. B. Sitosterol und Neomycin,
- Verbindungen, die über einen anderen Mechanismus als Hemmung der 2,3-Epoxisqualen-Lanosterol-Cyclase in die Cholesterolbiosynthese eingreifen, wie z. B. HMG-CoA-Reduktaseinhibitoren wie Lovastatin, Simvastatin, Pravastatin, Fluvastatin, Atorvastatin, Cerivastatin und andere,
- Squalen-Epoxidaseinhibitoren wie beispielsweise NB 598 und analoge Verbindungen sowie
- Squalen-Synthetaseinhibitoren wie beispielsweise Vertreter der Klasse der Isoprenoid-(phosphinylmethyl)phosphonate und Squalestatin.

Als weitere mögliche Kombinationspartner sind noch zu erwähnen die Klasse der Fibrate, wie Clofibrat, Bezafibrat, Gemfibrozil und andere, Nikotinsäure, ihre Derivate und Analoge wie beispielsweise Acipimox, sowie Probucol.

Desweiteren sind die Verbindungen der allgemeinen Formel I geeignet zur Behandlung von Erkrankungen, die mit überhöhter Zellproliferation im Zusammenhang stehen. Cholesterol ist ein essentieller Zellbestandteil und muß für die Zellproliferation, d. h. Zellteilung, in ausreichender Menge vorhanden sein. Die Inhibierung der Zellproliferation durch Inhibierung der Cholesterolbiosynthese ist am Beispiel der glatten Muskelzellen mit dem HMG-CoA-Reduktaseinhibitor des Mevinolintyps Lovastatin, wie eingangs erwähnt, beschrieben.

Als Beispiele für Erkrankungen, die mit überhöhter Zellproliferation zusammenhängen sind zunächst Tumorerkrankungen zu nennen. In Zellkultur- und in-vivo-Experimenten wurde gezeigt, daß die Senkung des Serumcholesterols oder der Eingriff in die Cholesterolbiosynthese durch HMG-CoA-Reduktaseinhibitoren das Tumorwachstum vermindert (Lancet 339, 1154-1156 [1992]). Die erfindungsgemäßen Verbindungen der Formel I sind deshalb aufgrund ihrer cholesterolbiosyntheseinhibitorischen Wirkung potentiell für die Behandlung von Tumorerkrankungen geeignet. Sie können dabei alleine oder zur Unterstützung bekannter Therapieprinzipien Verwendung finden.

Als weitere Beispiele sind hyperproliferative Hauterkrankungen wie beispielsweise Psoriasis, Basalzellkarzinome, Plattenepithelkarzinome, Keratosis und Keratinisierungsstörungen zu nennen. Der hier verwendete Ausdruck "Psoriasis" bezeichnet eine hyperproliferativ entzündliche Hauterkrankung, die den Regulierungsmechanismus der Haut verändert. Insbesondere werden Läsionen gebildet, die primäre und sekundäre Veränderungen der Proliferation in der Epidermis, entzündliche Reaktionen der Haut und die Expression regulatorischer Moleküle wie Lymphokine und Entzündungsfaktoren beinhalten. Psoriatische Haut ist morphologisch durch einen verstärkten Umsatz von Epidermiszellen, verdickte Epidermis, abnormale Keratinisierung entzündlicher Zellinfiltrate in die Dermisschicht und polymorphonucleäre Leukozyteninfiltration in die Epidermis, die eine Zunahme des Basalzellzyklus bedingt, gekennzeichnet. Zusätzlich sind hyperkeratotische und parakeratotische Zellen anwesend. Der Ausdruck "Keratosis", "Basalzellkarzinome", "Plattenepithelkarzinome" und "Keratinisierungsstörungen" bezieht sich auf hyperproliferative Hauterkrankungen, bei denen der Regulierungsmechanismus für die Proliferation und Differenzierung der Hautzellen unterbrochen ist.

Die Verbindungen der Formel I sind wirksam als Antagonisten der Hauthyperproliferation, d. h. als Mittel, die die Hyperproliferation menschlicher Keratinozyten hemmen. Die Verbindungen sind infolgedessen als Mittel zur Behandlung hyperproliferativer Hauterkrankungen wie Psoriasis, Basalzellkarzinomen, Keratinisierungsstörungen und Keratosis geeignet. Zur Behandlung dieser Krankheiten können die Verbindungen der Formel I entweder oral oder topisch appliziert werden, wobei sie entweder alleine in Form der Monotherapie oder in Kombination mit bekannten Wirkstoffen eingesetzt werden können.

Des weiteren zu nennen sind durch chirurgische Maßnahmen wie PTCA (perkutane transluminale coronare Angioplastie) oder Bypass-Operationen ausgelöste hyperproliferative Gefäßerkrankungen wie Stenosen und Gefäßverschlüsse, die auf der Proliferation glatter Muskelzellen beruhen. Wie eingangs erwähnt läßt sich diese Zellproliferation bekanntlich durch HMG-CoA-Reduktaseinhibitoren vom Mevinolintyp, wie Lovastatin, unterdrücken. Aufgrund ihrer inhibitorischen Wirkung auf die Cholesterolbiosynthese sind auch die Verbindungen der allgemeinen Formel I geeignet zur Behandlung und Prophylaxe dieser Erkrankungen, wobei sie entweder alleine oder in Kombination mit bekannten Wirkstoffen, wie z. B. intravenös appliziertes Heparin, vorzugsweise in oraler Applikation Verwendung finden können.

Eine weitere Einsatzmöglichkeit der erfindungsgemäßen Verbindungen der allgemeinen Formel I ist die Prophylaxe und Behandlung von Gallensteinleiden. Die Gallensteinbildung wird dadurch ausgelöst, daß die Cholesterolkonzentration in der Galle die maximale Löslichkeit des Cholesterols in der Gallenflüssigkeit überschreitet, wodurch es zur Ausfällung des Cholesterols in Form von Gallensteinen kommt. Lipidsenker aus der Klasse der Fibrate führen zu einer erhöhten Ausscheidung von Neutralsteroiden über die Galle und erhöhen die Neigung zur Gallensteinbildung.

Im Gegensatz dazu führen Cholesterolbiosynthesehemmer wie Lovastatin oder Pravastatin zu keiner erhöhten Gallensteinbildung, sondern können im Gegenteil eine Reduktion der Cholesterolkonzentration in der Galle bewirken und damit den sogenannten lithogenen Index, ein Maß für die Wahrscheinlichkeit der Gallensteinbildung, vermindern. Dies ist beschrieben in Gut 31, 348-350 [1990] sowie in Z. Gastroenterol. 29, 242-245 [1991].

Darüber hinaus ist in Gastroenterology 102, No. 4, Pt. 2, A 319 [1992] die Wirksamkeit von Lovastatin bei der Auflösung von Gallensteinen, insbesondere in Kombination mit Ursodeoxycholsäure beschrieben. Aufgrund ihrer Wirkungsweise sind die Verbindungen der allgemeinen Formel I deshalb auch für die Prophylaxe und Behandlung von Gallensteinleiden von Bedeutung. Sie können dabei entweder allein oder in Kombination mit bekannten Therapien wie beispielsweise der Behandlung mit Ursodeoxycholsäure oder der Schockwellenlithotripsie vorzugsweise in oraler Applikation Verwendung finden.

Schließlich sind die Verbindungen der allgemeinen Formel I geeignet zur Therapie von Infektionen durch pathogene Pilze wie z. B. Candida albicans, Aspergillus niger, Trichophyton mentagrophytes, Penicillium sp., Cladosporium sp. und andere. Wie bereits eingangs erwähnt ist das Endprodukt der Sterolbiosynthese im Pilzorganismus nicht Cholesterol, sondern das für die Integrität und Funktion der Pilzzellmembranen essentielle Ergosterol. Die Inhibierung der Ergosterolbiosynthese führt deshalb zu Wachstumsstörungen und gegebenenfalls zur Abtötung der Pilzorganismen.

Zur Behandlung von Mykosen können die Verbindungen der allgemeinen Formel I entweder oral oder topisch appliziert werden. Dabei können sie entweder alleine oder in Kombination mit bekannten antimykotischen Wirkstoffen eingesetzt werden, insbesondere mit solchen, die in andere Stufen der Sterolbiosynthese eingreifen, wie beispielsweise den Squalen-Epoxidasehemmern Terbinafin und Naftifin oder den Lanosterol-14α-Demethylaseinhibitoren vom Azol-Typ wie beispielsweise Ketoconazol und Fluconazol.

Eine weitere Verwendungsmöglichkeit der Verbindungen der allgemeinen Formel I betrifft die Anwendung in der Geflügelhaltung. Die Senkung des Cholesterolgehaltes von Eiern durch Verabreichung des HMG-CoA-Reduktaseinhibitors Lovastatin an Legehennen ist beschrieben (FASEB Journal 4, A 533, Abstracts 1543 [1990]). Die Erzeugung cholesterolarmer Eier ist von Interesse, da die Cholesterolbelastung des Körpers durch Eier mit reduziertem Cholesterolgehalt ohne eine Änderung der Ernährungsgewohnheiten vermindert werden kann. Aufgrund ihrer inhibitorischen Wirkung auf die Cholesterolbiosynthese können die Verbindungen der allgemeinen Formel I auch in der Geflügelzucht zur Erzeugung cholesterolarmer Eier Verwendung finden, wobei die Substanzen vorzugsweise als Zusatz zum Futter verabreicht werden.

Die biologische Wirkung von Verbindungen der allgemeinen Formel I wurde nach folgenden Methoden bestimmt:

### I. Messung der Hemmung des ¹⁴C-Acetat-Einbaus in die mit Digitonin fällbaren Steroide:

Die Untersuchung der Hemmwirkung wurde nach der in J. Lipid. Res. 37, 148-157 [1996] beschriebenen Methode bei Testkonzentrationen von 10⁻⁸ und 10⁻⁹ Mol/l durchgeführt.

Beispielhaft werden die Testergebnisse der folgenden Verbindungen (A) bis (M) der allgemeinen Formel I sowie der Vergleichssubstanzen (U), (V) und (W) bei diesen Testkonzentrationen angegeben:
(A) N-(Benzylthio)thiocarbonyl-4-[4-(dimethylaminomethyl)-phenylthio]piperidin-hydrochlorid,
(B) N- (Benzylthio) thiocarbonyl-4- [4- (dimethylaminomethyl)benzoyl]piperidin-hydrochlorid,
(C) N-Benzyloxycarbonyl-4-[4-(dimethylaminomethyl)phenylthio]-piperidin-hydrochlorid,
(D) N-(4-Chlorphenoxy)carbonyl-4-[4-(dimethylaminomethyl)-phenylthio]piperidin-hydrochlorid,
(E) N-(Benzylthio)thiocarbonyl-4-[4-(dimethylaminomethyl)-benzyl]piperidin-hydrochlorid,
(F) N-(Benzylthio)thiocarbonyl-4-[4-(dimethylaminomethyl)-benzyliden]piperidin-hydrochlorid,
(G) N-(4-Chlorphenoxy)thiocarbonyl-4-[4-(dimethylaminomethyl)-benzyl]piperidin-hydrochlorid,
(H) N-(4-Chlorphenoxy)carbonyl-4-[4-(dimethylaminomethyl)-benzyl]piperidin-hydrochlorid,
(I) N-Benzyloxycarbonyl-4-[4-(dimethylaminomethyl)benzyl]-piperidin-hydrochlorid,
(K) 4- [4- (Dimethylaminomethyl)benzyl] -N-(4-methylphenoxy)-carbonylpiperidin-hydrochlorid,
(L) 4- [4- (Dimethylaminomethyl)benzyl] -N- (4-methylphenoxy) - thiocarbonylpiperidin-hydrochlorid,
(M) 4-[4-(Dimethylaminomethyl)benzyl]-N-(4-fluorphenoxy)-carbonylpiperidin-hydrochlorid,
(U) 1-(4-Chlorbenzoyl)-4-[4-(2-oxazolin-2-yl)-benzyliden]-piperidin (EP-A-0 596 326, S. 16, dort Verbindung A; J. Lipid. Res. 38, 564-575 [1997]),
(V) trans-N-(4-Chlorbenzoyl)-N-methyl-[4-(4-dimethylamino)-methyl)phenyl]cyclohexylamin (DE-A-44 38 020; J. Lipid. Res. 37, 148-157 [1996]) und
(W) trans-O-(p-Tolylacetyl)-4-(4-dimethylaminomethylphenyl)-cyclohexanol (WO 95/29148, S. 28, dort Verbindung I).

Die Prozentwerte, um die obigen Verbindungen den ¹⁴C-Acetat-Einbau hemmen, sind in Tabelle 1 angegeben.

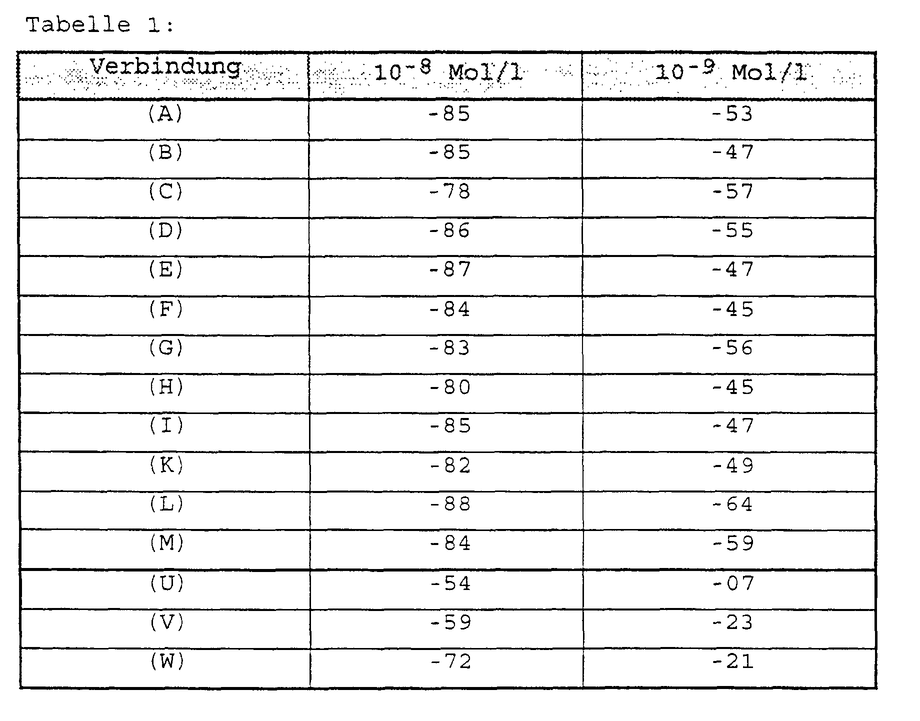

Von den Verbindungen H, I, K, und M wurden die IC₅₀-Werte bestimmt. Diese sind zusammen mit den IC₅₀-Werten der Verbindungen U, V und W in Tabelle 2 angegeben.

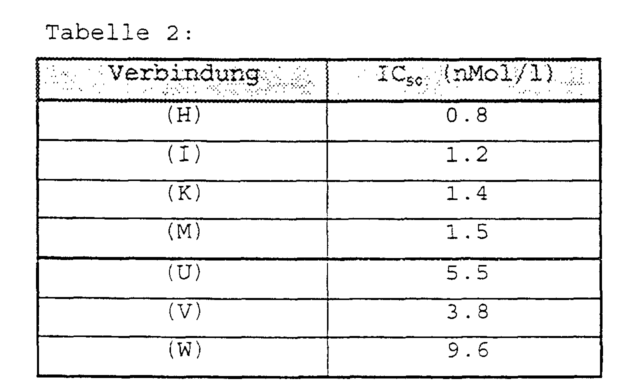

Aus Tabelle 2 ist eine signifikante Überlegenheit der erfindungsgemäßen Verbindungen gegenüber den vorbeschriebenen Vergleichssubstanzen ersichtlich.

### II. Messung der in-vivo Wirkung an der Ratte nach oraler Gabe

Die Inhibierung des Enzyms 2,3-Epoxisqualen-Lanosterol-Cyclase bewirkt eine Erhöhung der 2,3-Epoxisqualenspiegel in Leber und Plasma. Die Menge an gebildetem 2,3-Epoxisqualen dient daher als direktes Maß für die Wirkstärke am Ganztier.Die Bestimmung wurde nach der in J. Lipid. Res. 38, 564-575, [1997] beschriebenen Methode nach t = 3 bzw. 8 Stunden nach Substanzgabe mit Konzentrationen von c = 0.01, 0.03, 0.1, 0.3 und 1.0 mg/kg durchgeführt. In der folgenden Tabelle 3 sind beispielhaft die Ergebnisse für die vorstehend erwähnten Substanzen A, B, C, D, E, G, H, I und K angegeben.

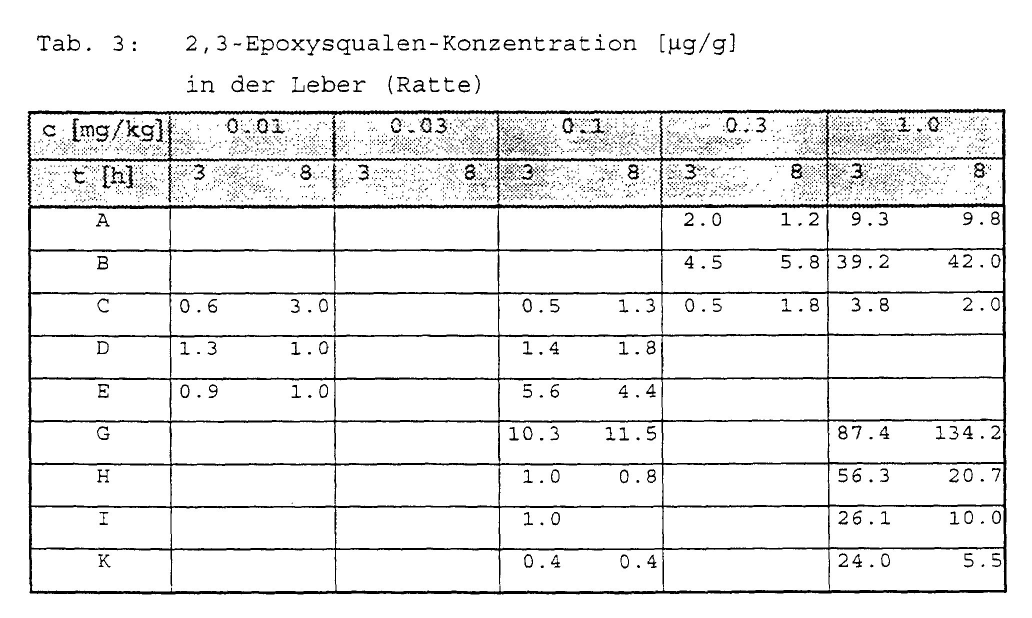

Bei den Kontrolltieren traten unter diesen Bedingungen keine messbaren 2,3-Epoxisqualenspiegel auf.

### III. Lipidsenkung am normolipämischen Goldhamster.

Diese Bestimmung wurde nach der in J. Lipid. Res. 38, 564-575, (1997) beschriebenen Methode durchgeführt. Am Ende des Versuchs wurde Gesamtcholesterol, β-Lipoprotein-Cholesterol sowie HDL-Cholesterol bestimmt und mit den Werten einer ohne Testsubstanz gefütterten Kontrollgruppe verglichen.

Geprüft wurde die lipidsenkende Wirkung der vorstehend erwähnten Verbindung H.

Die Ergebnisse sind in Tabelle 4 zusammengefaßt.

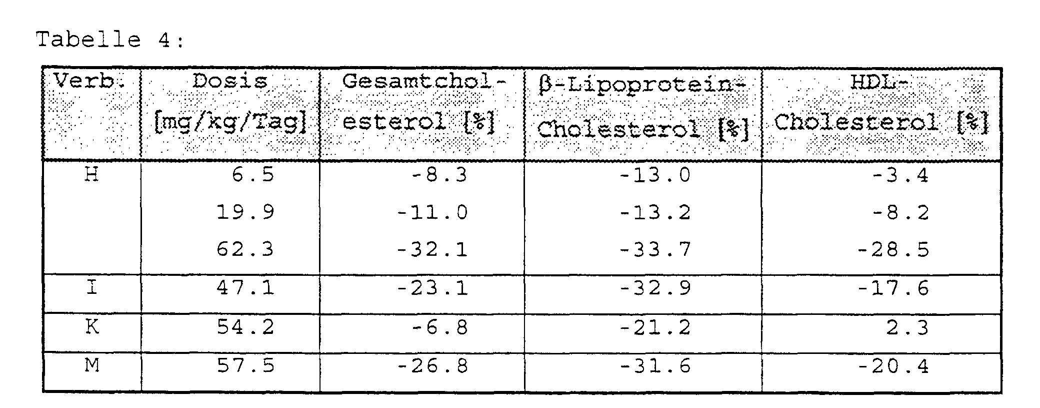

Unter diesen Bedingungen zeigten die Verbindungen keine toxischen Effekte.

### IV. Bestimmung der fungistatischen Wirkung

Die fungistatische Wirkung wurde über den Reihenverdünnungstest (Mikrotitersystem) bestimmt. Als Nährmedium diente Sabouraud-Bouillon. Das Inoculum betrug ca. 10⁴ bis 10⁵ KBE/ml (KBE = koloniebildende Einheiten); die Bebrütungszeit betrug 2 bis 4 Tage bei 26°C.

Es wurde die niedrigste Konzentration, die sichtbares Wachstum nicht mehr zuließ (minimale Hemmkonzentration MHK), bestimmt.

Geprüft wurden die vorstehend erwähnten Verbindungen A, B, D, E, F, H, I, K, L und M. Die Ergebnisse sind in der folgenden Tabelle 5 zusammengefaßt. Die MHK ist in µg/ml angegeben.

Folgende Testkeime wurden verwendet:

| Testkeim | Abkürzung |
|---|---|
| Cand. albicans ATCC 10231 | Cand. |
| Sacch. carlsbergensis ATCC 9080 | Sacc. |
| Rhod. rubra 49 | Rhod. |
| Asp. niger ATCC 16404 | Asp. |
| Trich. mentagrophytes ATCC 9129 | Trich. |
| Pen.notatum CBS 19746 | Pen. |

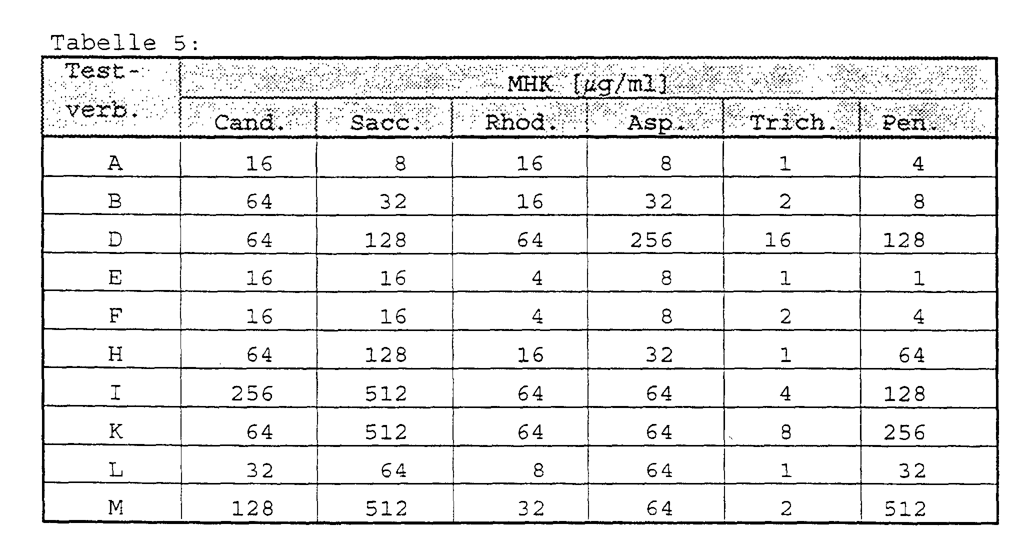

Zur pharmazeutischen Anwendung lassen sich die Verbindungen der allgemeinen Formel I in an sich bekannter Weise in die üblichen pharmazeutischen Zubereitungsformen für die orale, rektale und topische Verabreichung einarbeiten.

Formulierungen für die orale Verabreichung umfassen beispielsweise Tabletten, Dragées und Kapseln. Für die rektale Verabreichung kommen vorzugsweise Suppositorien in Betracht. Die Tagesdosis beträgt zwischen 0.1 und 200 mg für einen Menschen mit 60 kg Körpergewicht, bevorzugt ist jedoch eine Tagesdosis von 1 bis 100 mg für einen Menschen mit 60 kg Körpergewicht. Die Tagesdosis wird vorzugsweise in 1 bis 3 Einzelgaben aufgeteilt.

Bei topischer Anwendung können die Verbindungen in Zubereitungen, die etwa 1 bis 1000 mg, insbesondere 10 bis 300 mg Wirkstoff pro Tag enthalten, verabreicht werden. Die Tagesdosis wird vorzugsweise in 1 bis 3 Einzelgaben aufgeteilt.

Topische Formulierungen umfassen Gele, Cremes, Lotionen, Salben, Puder, Aerosole und andere herkömmliche Formulierungen zur Anwendung von Heilmitteln auf der Haut. Die Wirkstoffmenge für die topische Anwendung beträgt 1 bis 50 mg pro Gramm Formulierung, vorzugsweise jedoch 5 bis 20 mg pro Gramm Formulierung. Neben der Anwendung auf der Haut können die topischen Formulierungen der vorliegenden Erfindung auch angewandt werden bei der Behandlung von Schleimhäuten, die der topischen Behandlung zugänglich sind. Beispielsweise können die topischen Formulierungen auf die Schleimhäute des Mundes, des unteren Colons und andere aufgebracht werden.

Zur Anwendung in der Geflügelzucht zur Erzeugung cholesterolarmer Eier werden die Wirkstoffe der allgemeinen Formel I den Tieren nach den üblichen Methoden als Zusatz zu geeigneten Futtermitteln verabreicht. Die Konzentration der Wirkstoffe im Fertigfutter beträgt normalerweise 0.01 bis 1%, vorzugsweise jedoch 0.05 bis 0.5%.

Die Wirkstoffe können als solche dem Futter zugesetzt werden. So enthalten die erfindungsgemäßen Futtermittel für Legehennen neben dem Wirkstoff und gegebenenfalls neben einer üblichen Vitamin-Mineral-Mischung beispielsweise Mais, Sojabohnenmehl, Fleischmehl, Futterfett und Sojaöl. Zu diesem Futter wird eine der eingangs erwähnten Verbindungen der Formel I als Wirkstoff in einer Konzentration von 0.01 bis 1%, vorzugsweise jedoch 0.05 bis 0.5% zugemischt.

Die nachfolgenden Beispiele dienen der näheren Erläuterung der Erfindung. Die angegebenen R_{f}-Werte wurden an Fertigplatten der Firma E.Merck, Darmstadt bestimmt und zwar an:
a) Aluminiumoxid F-254 (Typ E)
b) Kieselgel 60 F-254

### Beispiele zur Herstellung der Ausgangsmaterialien:

### Beispiel A

### 4-[4-(Dimethylaminomethyl)phenylthio]piperidin

Zu einer Lösung von 7.18 g ( 71 mmol) 4-Hydroxypiperidin in 100 ml Dimethylformamid werden zuerst 19.6 g (142 mmol) gepulvertes Kaliumcarbonat gegeben und anschliessend 19.8 g (71 mmol) Tritylchlorid zugefügt. Es wird 22 Stunden bei Raumtemperatur gerührt, mit Essigsäureethylester auf das doppelte Volumen verdünnt, mit Wasser und gesättigter Kochsalzlösung gewaschen, mit Magnesiumsulfat getrocknet und eingedampft. Der Rückstand wird aus Essigsäureethylester/Petrolether (1:4, v:v) umkristallisiert. Man erhält 17.65 g (72.4 % d.Th.) 4-Hydroxy-N-tritylpiperidin als farblose Kristalle vom Schmelzpunkt 157-158°C.

5.77 g dieses Produkts werden in 60 ml Methylenchlorid gelöst, 2.6 g Methansulfonsäurechlorid zugefügt und 3.4 g Triethylamin langsam zugetropft. Es wird 1 Stunde bei 0°C nachgerührt, mit Ether verdünnt, mit Eiswasser gewaschen (4x), mit Magnesiumsulfat getrocknet und eingedampft. Man erhält 7.58 g 4-Methansulfonyloxy-N-tritylpiperidin als farblosen Schaum.

Das Produkt wird in 30 ml Tetrahydrofuran gelöst und zu einer Lösung des Natriumsalzes von 4-Bromthiophenol (hergestellt aus 3.18 g 4-Bromthiophenol und 0.81 g 55%-igem Natriumhydrid in 35 ml Tetrahydrofuran) zugetropft. Es wird 1 Stunde zum Sieden erhitzt, nach Abkühlen in Essigsäureethylester aufgenommen, mit Wasser und gesättigter Kochsalzlösung gewaschen, mit Magnesiumsulfat getrocknet und eingedampft. Man erhält 5.28 g 4-(4-Bromphenylthio)-N-tritylpiperidin als farblose Kristalle vom Schmelzpunkt 174-175°C.

2.57 g (5mmol) dieses Produkts werden in 30 ml Tetrahydrofuran gelöst und bei -70°C 4 ml (6.4mmol) einer 1.6-molaren Lösung von n-Butyllithium in n-Hexan zugetropft. Nach 1.5 Stunden bei -70°C werden 1.1 g (6.4 mmol) N,N-Dimethyl-methylenimmoniumjodid zugegeben, das Kühlbad entfernt und über Nacht gerührt. Das Lösungsmittel wird verdampft, der Rückstand mit Wasser verrieben und mit Essigsäureethylester extrahiert. Die organische Phase wird mit Magnesiumsulfat getrocknet, eingedampft und der Rückstand durch Säulenchromatographie (Aluminiumoxid, Essigsäureethylester/Petrolether = 1:10, v:v) gereinigt. Man erhält 0.9 g 4-[4-(Dimethylaminomethyl)phenylthio]-N-tritylpiperidin als farblose Kristalle vom Schmelzpunkt 163°C.

Dieses Produkt wird in 30 ml Methylenchlorid gelöst und 10 ml etherische Salzsäure zugegeben. Nach 1 Stunde bei Raumtemperatur wird eingeengt, mit Ether versetzt und der Niederschlag abgesaugt. Dieser wird in wenig Wasser gelöst, Ether zugegeben und mit 6N-Natronlauge auf pH 11 eingestellt. Die Etherphase wird abgetrennt, die Wasserphase nochmals mit Ether extrahiert und die vereinigten Extrakte mit Magnesiumsulfat getrocknet. Man erhält 500 mg 4-[4-(Dimethylaminomethyl)phenylthio]piperidin als farblose Kristalle vom Schmelzpunkt 52°C.

### Beispiel B

### 4-[4-(Piperidinomethyl)phenylthio]piperidin

10.1 g (0.1 Mol) 4-Hydroxypiperidin und 11 g (0.11 Mol) Triethylamin werden in 200 ml Essigsäureethylester und 250 ml Tetrahydrofuran vorgelegt und eine Lösung von 23.3 g (0.11 Mol) Chlorameisensäure-2.2.2-trichlorethylester bei 0-4°C zugetropft. Es wird 1 Stunde bei 0°C und 1 Stunde bei Raumtemperatur nachgerührt. Der Niederschlag wird abgesaugt, das Filtrat bei -12 bis -14°C mit 12 g (0.105 Mol) Methansulfonsäurechlorid versetzt und bei -8 bis -12°C 12 g (0.12 Mol) Triethylamin in 50 ml Tetrahydrofuran zugetropft. Es wird 40 Minuten bei -10°C gerührt, das Kühlbad entfernt und 30 Minuten nachgerührt. Nach Waschen mit Wasser und gesättigter Kochsalzlösung wird mit Magnesiumsulfat getrocknet, eingedampft, der Rückstand mit Diisopropylether verrieben und abgesaugt. Man erhält 32.4 g (91.5 % d.Th.) 4-Methansulfonyloxy-N-2.2.2-trichlorethoxycarbonylpiperidin als farblose Kristalle vom Schmelzpunkt 93°C.

17.7 g (50 mmol) dieses Produkts in 30 ml Dimethylformamid werden bei Raumtemperatur zu einer Lösung von Kaliumthiophenolat (hergestellt aus 6 g Thiophenol und 6.2 g Kalium-tert.butylat in 50 ml Dimethylformamid bei 20-60°C) getropft. Die entstehende Gallerte wird mit 100 ml Dimethylformamid versetzt, auf 60°C erwärmt und nach Zugabe von 30 ml Methanol über Nacht bei Raumtemperatur stehengelassen. Nach Zugabe von 800 ml Wasser wird mit Ether extrahiert, der Etherextrakt mit Wasser gewaschen, mit Magnesiumsulfat getrocknet und eingedampft. Nach Reinigen durch Säulenchromatographie (Kieselgel, Petrolether/Essigsäureethylester = 5:1, v:v) erhält man 11 g (64.3 % d.Th.) 4-Phenylthio-N-2.2.2-trichlorethoxycarbonylpiperidin als farbloses Öl.

9 g (25 mmol) dieses Produkts, 6 g Paraformaldehyd und 5.6 g (42 mmol) Zinkchlorid werden in 300 ml Methylenchlorid vorgelegt. Bei 20-22°C wird 30 Minuten lang Chlorwasserstoff eingeleitet. Nach einer Stunde wird nochmals 10 Minuten lang Chlorwasserstoff eingeleitet und dann über Nacht gerührt. Die Reaktionsmischung wird in 300 ml einer 1-molaren Dinatriumhydrogenphosphatlösung eingerührt, die Methylenchloridphase abgetrennt und die Wasserphase mit Methylenchlorid extrahiert. Die organischen Phasen werden vereinigt, mit Wasser gewaschen, mit Magnesiumsulfat getrocknet und eingedampft. Nach Reinigung durch Säulenchromatographie (Kieselgel, Essigsäureethylester/Petrolether = 1:10, v:v) erhält man 4.5 g (43.2 % d.Th.) 4-[4-(Chlormethyl)phenylthio]-N-2.2.2-trichlorethoxycarbonylpiperidin als farbloses Öl.

2.1 g ( 5mmol ) dieses Produkts und 1.3 g (15 mmol) Piperidin werden in 10 ml Tetrahydrofuran und 10 ml Ethanol 2,5 Stunden zum Rückfluss erhitzt. Nach Verdampfen wird mit Wasser verrieben und mit Ether extrahiert.Die Etherphase wird mit Wasser gewaschen, mit Magnesiumsulfat getrocknet und eingedampft. Man erhält 2.5 g 4-[4-(Piperidinomethyl)phenylthio]-2.2.2-trichlorethoxycarbonylpiperioin als Rohprodukt.

2.4 g dieses Produkts werden in 3.5 ml Essigsäure und 18 ml Wasser gelöst und 5 g Zinkpulver zugegeben (heftiges Schäumen). Es wird 20 Stunden bei Raumtemperatur und 1 Stunde bei 50 °C gerührt. Nach Zugabe von 20 ml Wasser wird mit 100 ml Ether überschichtet, mit 6N-Natronlauge stark alkalisch gestellt und 20 Minuten gerührt. Der Ether wird abgetrennt und die Wasserphase 3 x mit Ether extrahiert. Die vereinigten Extrakte werden mit Magnesiumsulfat getrocknet und eingedampft. Man erhält 1.3 g 4-[4-(Piperidinomethyl)phenylthio]piperidin als farbloses Pulver.
R_{f}-Wert: 0.69 (Aluminiumoxid,Methylenchlorid/Methanol = 10:1, v:v).

### Beispiel C

### 4-[4-(Dimethylaminomethyl)benzyl]piperidin

100 g (0.57 Mol) 4-Benzylpiperidin in 220 ml Methanol werden bei 20 bis 35°C mit 88 g (0.68 Mol) Trifluoressigsäuremethylester versetzt. Nach 2 Stunden wird eingedampft. Nach Kristallisation des Rückstands aus Petrolether erhält man 117 g (76 % d.Th.) 4-Benzyl-N-trifluoracetylpiperidin als farblose Kristalle.

113 g (0.848 Mol) Aluminiumchlorid in 600 ml Dichlorethan werden mit 114 g (0.9 Mol) Oxalylchlorid versetzt. Bei -4 bis +4°C werden 114 g des obigen Produkts in 300 ml Dichlorethan zugetropft (Gasentwicklung). Es wird 2.5 Stunden bei Raumtemperatur gerührt und anschliessend bei -25°C 300 ml einer 40%igen wässrigen Dimethylaminlösung rasch zugegeben (Temperaturanstieg bis 35°C). Die entstandene Gallerte wird mit 100 ml Dichlorethan verdünnt, 20 Minuten gerührt und zur besseren Phasentrennung mit Chloroform verdünnt. Es wird mit Wasser, 2N-Natronlauge, Wasser, 2N-Salzsäure und nochmals mit Wasser gewaschen. Die organische Phase wird mit Magnesiumsulfat getrocknet, eingedampft und der Rückstand mit Ether verrieben. Man erhält 111 g (80 % d.Th.) 4-[4-(Dimethylaminocarbonyl)-benzyl]-N-trifluoracetylpiperidin als farblose Kristalle.

64 g dieses Produkts in 350 ml Tetrahydrofuran werden bei 0 bis 3°C zu 70 ml einer 20 %-igen Lösung von Lithiumaluminiumhydrid in Ether, verdünnt mit 250 ml Ether, getropft. Es wird 20 Minuten bei 0°C gerührt und anschliessend 1 Stunde zum Rückfluss erhitzt. Bei 0 bis 15°C werden 40 ml 4N-Natronlauge zugetropft (starkes Schäumen), 30 Minuten bei Raumtemperatur gerührt, abgesaugt und der Filterrückstand mit Ether gewaschen. Die vereinigten Etherphasen werden mit Magnesiumsulfat getrocknet und eingedampft. Man erhält 46 g (99 % d.Th.) 4-[4-(Dimethylaminomethyl)benzyl]piperidin als farbloses, langsam kristallisierendes Öl.
R_{f}-Wert: 0.56 (Aluminiumoxid, Methylenchlorid/Methanol = 10:1, v:v).

### Beispiele zur Herstellung der Endprodukte:

### Beispiel 1

### N-(Benzylthio)thiocarbonyl-4-[4-(dimethylaminomethyl)phenylthio]piperidin-hydrochlorid

Ein Gemisch aus 237 mg (0.83 mmol) 4-[4-(Dimethylaminomethyl)-phenylthio]piperidin, 200 mg (2mmol) Triethylamin und 1 ml Ethanol werden bei Raumtemperatur mit 150 mg (2 mmol) Schwefelkohlenstoff versetzt. Der entstehende Niederschlag wird durch Zugabe von 1 ml Triethylamin und 2.5 ml Dimethylformamid in Lösung gebracht. Nach 1 Stunde bei Raumtemperatur werden 160 mg (0.94 mmol) Benzylbromid zugegeben, über Nacht bei Raumtemperatur gerührt und anschließend 3 Stunden auf 60°C erwärmt. Nach Zugabe von 50 ml Wasser wird mit Essigsäureethylester extrahiert, die organische Phase mit Magnesiumsulfat getrocknet und eingedampft. Nach Reinigung des Rückstands durch Säulenchromatographie (Aluminiumoxid, Petrolether/Essigsäureethylester = 4:1, v:v) erhält man 210 mg der Titelverbindung als farbloses Öl, das mit etherischer Salzsäure in das Hydrochlorid überführt wird.
R_{f}-Wert der freien Base: 0.83 (Aluminiumoxid, Essigsäureethylester/Petrolether = 3:1, v:v).
¹H-NMR-Spektrum (200 MHz,DMSO-d₆), Signale bei ppm: 1.4-1.6 (m, 2H), 2.0-2.15 (m, 2H), 2.55 (d, 6H), 3.5-3.65 (t, 2H), 3.7-3.85 (m, 1H ), 4.2 (d, 2H), 4.3-4.4 (m, 1H), 4.5 (s, 2H), 4.95-5.15 (m, 1H), 7.2-7.6 (m, 9H)

Analog werden erhalten:
(1) N-(Benzylthio)thiocarbonyl-4-[4-(dimethylaminomethyl)-benzoyl]piperidin-hydrochlorid,
   aus 4-[4-(Dimethylaminomethyl)benzoyl]piperidin und Benzylbromid; farbloses Pulver; R_{f}-Wert der freien Base: 0.65 (Aluminiumoxid, Essigsäurethylester)
(2) N-(Benzylthio)thiocarbonyl-4-[4-(dimethylaminomethyl)-benzyliden]piperidin-hydrochlorid,
   aus 4-[4-(Dimethylaminomethyl)benzyliden]piperidin und Benzylbromid; farbloses Pulver; Schmelzpunkt: 190°C
(3) N-(Benzylthio)thiocarbonyl-4-[4-(dimethylaminomethyl)-benzyl]piperidin-hydrochlorid,
   aus 4-[4-(Dimethylaminomethyl)benzyl]piperidin und Benzylbromid; farbloses Pulver; R_{f}-Wert der freien Base: 0.26 (Aluminiumoxid, Petrolether/Essigsäureethylester = 10:1, v:v)

### Beispiel 2

### N-Benzyloxycarbonyl-4-[4-(dimethylaminomethyl)phenylthio]-piperidin

250 mg (1mmol) 4-[4-(Dimethylaminomethyl)phenylthio]piperidin in 20 ml Tetrahydrofuran werden mit 2 ml 2N-Natronlauge und 5 ml Wasser versetzt. Bei Raumtemperatur werden 200 mg (1.2 mmol) Chlorameisensäurebenzylester in 5 ml Tetrahydrofuran langsam zugetropft. Nach 1.5 Stunden bei Raumtemperatur werden nochmals etwas Chlorameisensäurebenzylester und Natronlauge zugegeben. Nach Zugabe von 100 ml Ether wird mit gesättigter Kochsalzlösung gewaschen, die organische Phase mit Magnesiumsulfat getrocknet und eingedampft. Das nach Reinigung durch Säulenchromatographie (Aluminiumoxid, Petrolether/Essigsäureethylester = 4:1, v:v) erhaltene Produkt wird mit etherischer Salzsäure in das Hydrochlorid überführt. Man erhält 190 mg (45.1 % d. Th.) der Titelverbindung als farbloses Pulver vom Schmelzpunkt 144°C.
¹H-NMR-Spektrum (200 MHz,DMSO-d₆), Signale bei ppm:
1.3-1.5 (m, 2H), 1.85-2.0 (m, 2H), 2.65 (s, 6H), 2.95-3.15 (t, 2H), 3.5-3.65 (m, 1H ), 3.8-4.0 (m, 2H), 4.2 (s, 2H), 5.05 (s, 2H), 7.3-7.6 (m, 9H)

Analog werden erhalten:
(1) N-(4-Chlorphenylthio)thiocarbonyl-4-[4-(dimethylaminomethyl)phenylthio]piperidin-hydrochlorid,
   aus 4-[4-(Dimethylaminomethyl)phenylthio]piperidin und Chlordithioameisensäure-4-chlorphenylester; farbloses Pulver; Schmelzpunkt: 178°C
(2) 4-[4-(Dimethylaminomethyl)phenylthio]-N-phenoxycarbonylpiperidin-hydrochlorid,
   aus 4-[4-(Dimethylaminomethyl)phenylthio]piperidin und Chlorameisensäurephenylester; farbloses Pulver;
   Schmelzpunkt: 161°C
(3) N-(4-Chlorphenoxy)carbonyl-4-[4-(dimethylaminomethyl)-phenylthio]piperidin-hydrochlorid,
   aus 4-[4-(Dimethylaminomethyl)phenylthio]piperidin und Chlorameisensäure-4-chlorphenylester; farbloses Pulver;
   Schmelzpunkt:169°C
(4) 4-[4-(Dimethylaminomethyl)phenylthio]-N-(phenylthio)thiocarbonylpiperidin-hydrochlorid,
   aus 4-[4-(Dimethylaminomethyl)phenylthio]piperidin und Chlordithioameisensäurephenylester; farbloses Pulver;
   R_{f}-Wert der freien Base: 0.57 (Aluminiumoxid, Petrolether/Essigsäureethylester = 4:1, v:v)
(5) N-4-(4-Chlorphenoxy)thiocarbonyl-4-[4-(dimethylaminomethyl)benzyl]piperidin-hydrochlorid,
   aus 4-[4-(Dimethylaminomethyl)benzyl]piperidin und Chlorthioameisen-O-4-chlorphenylester; farbloses Pulver;
   Schmelzpunkt :150°C
(6) N-(4-Chlorphenoxy)carbonyl-4-[4-(dimethylaminomethyl)-benzyl]piperidin,
   aus 4-[4-(Dimethylaminomethyl)benzyl]piperidin und Chlorameisensäure-4-chlorphenylester;
   Schmelzpunkt der freien Base: 104°C;
   Durch Behandeln mit Salzsäure wurde das Hydrochlorid erhalten. Farbloses Pulver; Schmelzpunkt: 156°C;
   Durch Behandeln der freien Base mit Methansulfonsäure in einem Essigsäureethylester-Ether-Gemisch wurde das Methansulfonat erhalten. Farbloses Pulver; Schmelzpunkt: 152°C.
   Durch Behandeln der freien Base mit L-Weinsäure in einem Methanol-Essigsäureethylester-Gemisch wurde das Tartrat erhalten. Farbloses Pulver; Schmelzpunkt: 147°C
(7) N-(4-Chlorphenylthio)carbonyl-4-[4-(dimethylaminomethyl)-benzyl]piperidin-hydrochlorid,
   aus 4-[4-(Dimethylaminomethyl)benzyl]piperidin und Chlorthioameisensäure-S-4-chlorphenylester; farbloses Pulver;
   Schmelzpunkt: 190-191°C
(8) N-Benzyloxycarbonyl-4-[4-(piperidinomethyl)phenylthio]-piperidin-hydrochlorid,
   aus 4-[4-(Piperidinomethyl)phenylthio]piperidin und Chlorameisensäurebenzylester; farblosesPulver; Schmelzpunkt: 186°C
(9) N-Phenoxycarbonyl-4-[4-(piperidinomethyl)phenylthio]-piperidin-hydrochlorid,
   aus 4-[4-(Piperidinomethyl)phenylthio]piperidin und Chlorameisensäurephenylester; farbloses Pulver;
   Schmelzpunkt: 204°C
(10) N-4-Chlorphenoxycarbonyl-4-[4-(piperidinomethyl)phenylthio]piperidin-hydrochlorid,
   aus 4-[4-(Piperidinomethyl)phenylthio]piperidin und Chlorameisensäure-4-chlorphenylester; farbloses Pulver;
   Schmelzpunkt: 182°C
(11) N-Benzyloxycarbonyl-4-[4-(dimethylaminomethyl)benzyl]-piperidin,
   aus 4-[4-(Dimethylaminomethyl)benzyl]piperidin und Chlorameisensäurebenzylester;
   Schmelzpunkt der freien Base: 63°C;
   Durch Behandeln mit Salzsäure wurde das Hydrochlorid erhalten. Farbloses Pulver; Schmelzpunkt: 132°C;
   Durch Behandeln der freien Base mit Methansulfonsäure in einem Essigsäureethylester-Ether-Gemisch wurde das Methansulfonat erhalten. Farbloses Pulver; Schmelzpunkt: 140°C;
   Durch Behandeln der freien Base mit L-Weinsäure in einem Methanol-Essigsäureethylester-Gemisch wurde das Tartrat erhalten. Farbloses Pulver; Schmelzpunkt: 125°C
(12) 4-[4-(Dimethylaminomethyl)benzyl]-N-(4-methylphenoxy)carbonylpiperidin,
   aus 4-[4-(Dimethylaminome yl)benzyl]piperidin und Chlorameisensäure-4-methylphenylester;
   Schmelzpunkt der freien Base: 80°C;
   Durch Behandeln mit Salzsäure wurde das Hydrochlorid erhalten. Farbloses Pulver; Schmelzpunkt: 185°C;
   Durch Behandeln der freien Base mit Methansulfonsäure in einem Essigsäureethylester-Ether-Gemisch wurde das Methansulfonat erhalten. Farbloses Pulver; Schmelzpunkt: 165°C;
   Durch Behandeln der freien Base mit L-Weinsäure in einem Methanol-Essigsäureethylester-Gemisch wurde das Tartrat erhalten. Farbloses Pulver; Schmelzpunkt: 162°C
(13) 4-[4-(Dimethylaminomethyl)benzyl]-N-(4-methylphenoxy)-thiocarbonylpiperidin-hydrochlorid,
   aus 4-[4-(Dimethylaminomethyl)benzyl]piperidin und Chlorthioameisensäure-O-4-methylphenylester; farbloses Pulver; Schmelzpunkt: 184°C
(14) 4-[4-(Dimethylaminomethyl)benzyl]-N-(4-fluorphenoxy)carbonylpiperidin-hydrochlorid,
   aus 4-[4-(Dimethylaminomethyl)benzyl]piperidin und Chlorameisensäure-4-fluorphenylester; farbloses Pulver;
   R_{f}-Wert der freien Base: 0.61 (Aluminiumoxid, Petrolether/Essigsäureethylester = 6:1, v:v)

### Beispiel 3

### N-Benzyloxycarbonyl-4-[4-(dimethylaminomethyl)phenylsulfinyl]piperidin-hydrochlorid

90.7 mg (0.215 mmol) N-Benzyloxy-4-[4-(dimethylaminomethyl)-phenylthio]piperidin-hydrochlorid werden in einer Mischung aus 1 ml Methanol und 1 ml Wasser gelöst und es werden zuerst 18.6 mg (0.226 mmol) wasserfreies Natriumacetat und dann 48.4 mg (0.226 mmol) Natriummetaperjodat zugefügt. Es wird 6 Stunden bei Raumtemperatur gerührt, mit Wasser verdünnt, mit Essigsäureethylester überschichtet und mit Soda gesättigt. Die organische Phase wird abgetrennt, die Wasserphase mit Essigsäureethylester extrahiert, die organischen Phasen vereinigt, mit gesättigter Kochsalzlösung gewaschen, mit Magnesiumsulfat getrocknet und eingedampft. Der Rückstand wird in wenig Methylenchlorid gelöst, mit etherischer Salzsäure versetzt und das Lösungsmittel verdampft. Der Rückstand wird mit Ether verrieben, das Lösungsmittel verdampft und der Rückstand im Hochvakuum getrocknet. Man erhält 85 mg (90.5 % d.Th.) der Titelverbindung als farbloses Pulver.
R_{f}-Wert der freien Base: 0.43 (Aluminiumoxid, Essigsäureethylester/Petrolether = 1:1, v:v).
¹H-NMR-Spektrum (200 MHz,DMSO-d₆), Signale bei ppm: 1.3-1.6 (m, 3H), 1.8-2.0 (m, 1H), 2.65 (s, 6H), 2.7-3.1 (m, 3H), 3.95-4.15 (t, 2H), 4.35 (s, 2H), 5.05 (s, 2H), 7.3 (s, 5H), 7.65-7.85 (q, 4H)

Analog wird erhalten:
(1) 4-[4-(Dimethylaminomethyl)phenylsulfinyl]-N-phenoxycarbonylpiperidin-hydrochlorid,
   aus 4-[4-(Dimethylaminomethyl)phenylthio]-N-phenoxycarbonylpiperidin-hydrochlorid und Natriummetaperjodat; farbloses Pulver; R_{f}-Wert der freien Base: 0.4 (Aluminiumoxid, Essigsäureethylester/Petrolether = 1:1, v:v).

## Patentansprüche

1. Von Azacycloalkanen abgeleiteten Urethane und deren Thiound Dithioanaloga der allgemeinen Formel 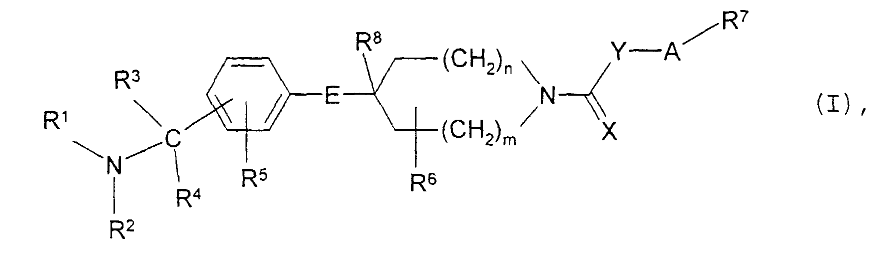 in der
m die Zahlen 0 oder 1,
n die Zahlen 1 oder 2,
A eine Einfachbindung, eine geradkettige oder verzweigte C₁₋₈-Alkylengruppe, eine C₂₋₈-Alkenylen- oder C₂₋₈-Alkinylengruppe, wobei eine ungesättigte Gruppe nicht direkt an den Rest Y gebunden ist,
X ein Sauerstoff- oder Schwefelatom,
Y ein Sauerstoff- oder Schwefelatom,
R¹ eine geradkettige oder verzweigte C₁₋₆-Alkylgruppe, eine C₁₋₄-Alkenylgruppe oder eine C₁₋₄-Alkinylgruppe, wobei die Mehrfachbindung von der Stickstoff-Kohlenstoff-Bindung isoliert ist,
R² eine geradkettige oder verzweigte C₁₋₆-Alkylgruppe, eine C₁₋₄-Alkenyl- oder C₁₋₄-Alkinylgruppe, wobei eine Mehrfachbindung von der Stickstoff-Kohlenstoff-Bindung isoliert ist, oder
R¹ und R² zusammen mit dem Stickstoffatom einen 5-bis 7-gliedrigen, gesättigten heterocyclischen Ring, in dem eine von dem Stickstoffatom isolierte Methylengruppe durch ein Sauerstoffoder Schwefelatom oder durch eine -NH- oder -N(Alkyl)-Gruppe ersetzt sein kann,
R³ bis R⁶, die gleich oder verschieden sein können, Wasserstoffatome oder Alkylgruppen,
R⁷ eine C₃₋₇-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe oder, sofern A keine Einfachbindung darstellt, auch ein Wasserstoffatom,
E ein Sauerstoff- oder Schwefelatom, eine Methylen-, Carbonyloder Sulfinylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten, wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
deren Enantiomere, Diastereomere, deren Gemische und deren Salze.

2. Verbindungen der allgemeinen Formel I gemäß Anspruch 1, in der
m die Zahl 1,
n die Zahl 1,
A eine Einfachbindung, eine geradkettige oder verzweigte C₁₋₄-Alkylengruppe oder eine C₂₋₄-Alkenylengruppe, wobei eine ungesättigte Gruppe nicht direkt an den Rest Y gebunden ist,
X ein Sauerstoff- oder Schwefelatom,
Y ein Sauerstoff- oder Schwefelatom,
R¹ eine geradkettige oder verzweigte C₁₋₆-Alkylgruppe, eine Allyl- oder Propargylgruppe, wobei die Mehrfachbindung von der Stickstoff-Kohlenstoff-Bindung isoliert ist,
R² eine geradkettige oder verzweigte C₁₋₆-Alkylgruppe, eine Allyl- oder Propargylgruppe, wobei die Mehrfachbindung von der Stickstoff-Kohlenstoff-Bindung isoliert ist, oder
R¹ und R² zusammen mit dem Stickstoffatom einen 5- bis 7-gliedrigen, gesättigten heterocyclischen Ring, in dem eine von dem Stickstoffatom isolierte Methylengruppe durch ein Sauerstoffoder Schwefelatom ersetzt sein kann,
R³ bis R⁶, die gleich oder verschieden sein können, Wasserstoffatome oder Methylgruppen,
R⁷ eine C₃₋₆-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe,
E ein Schwefelatom, eine Methylen-, Carbonyl- oder Sulfinylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten, wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
deren Enantiomere, Diastereomere, deren Gemische und deren Salze.

3. Verbindungen der allgemeinen Formel I gemäß Anspruch 1, in der
m die Zahl 1,
n die Zahl 1,
A eine Einfachbindung oder eine geradkettige oder verzweigte C₁₋₃-Alkylengruppe,
X ein Sauerstoff- oder Schwefelatom,
Y ein Sauerstoff- oder Schwefelatom,
R¹ eine geradkettige oder verzweigte C₁₋₃-Alkylgruppe,
R² eine geradkettige oder verzweigte C₁₋₃-Alkylgruppe oder
R¹ und R² zusammen mit dem Stickstoffatom eine Piperidino- oder Morpholinogruppe,
R³ bis R⁶ Wasserstoffatome,
R⁷ eine Cyclohexylgruppe oder eine gegebenenfalls durch ein Halogenatom, durch eine Alkyl-, Alkoxy- oder Trifluormethylgruppe substituierte Phenylgruppe,
E ein Schwefelatom, eine Methylengruppe, eine Carbonyl- oder Sulfinylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten,
deren Enantiomere, Diastereomere, deren Gemische und deren Salze.

4. Verbindungen der allgemeinen Formel I gemäß Anspruch 1, in der
m die Zahl 1,
n die Zahl 1,
A eine Einfachbindung oder eine Methylengruppe,
X ein Sauerstoff- oder Schwefelatom,
Y ein Sauerstoff- oder Schwefelatom,
R¹ und R² jeweils eine Methylgruppe,
R³ bis R⁶ Wasserstoffatome,
R⁷ eine gegebenenfalls durch ein Fluor- oder Chloratom oder durch eine Methylgruppe substituierte Phenylgruppe,
E ein Schwefelatom, eine Methylen- oder Carbonylgruppe und
R⁸ ein Wasserstoffatom oder
E die Gruppe -C(R⁹R¹⁰)-, wobei
R⁹ ein Wasserstoffatom und
R¹⁰ zusammen mit der benachbarten Gruppe R⁸ eine Kohlenstoff-Kohlenstoff-Bindung darstellt,
bedeuten, deren Gemische und deren Salze.

5. Folgende Verbindungen der allgemeinen Formel I gemäß Anspruch 1:
(1) N-(Benzylthio)thiocarbonyl-4-[4-(dimethylaminomethyl)-phenylthio]piperidin,
(2) N-(Benzylthio)thiocarbonyl-4-[4-(dimethylaminomethyl)benzoyl]piperidin,
(3) N-Benzyloxycarbonyl-4-[4-(dimethylaminomethyl)phenylthio]-piperidin,
(4) N-(4-Chlorphenoxy)carbonyl-4-[4-(dimethylaminomethyl)phenylthio]piperidin,
(5) N-(Benzylthio)thiocarbonyl-4-[4-(dimethylaminomethyl)benzyl]piperidin,
(6) N-(Benzylthio)thiocarbonyl-4-[4-(dimethylaminomethyl)benzyliden]piperidin,
(7) N-(4-Chlorphenoxy)thiocarbonyl-4-[4-(dimethylaminomethyl)-benzyl]piperidin,
(8) N-(4-Chlorphenoxy)carbonyl-4-[4-(dimethylaminomethyl)-benzyl]piperidin,
(9) N-Benzyloxycarbonyl-4-[4-(dimethylaminomethyl)benzyl]-piperidin,
(10) 4-[4-(Dimethylaminomethyl)benzyl]-N-(4-methylphenoxy)-carbonylpiperidin,
(11) 4- [4-(Dimethylaminomethyl)benzyl]-N-(4-methylphenoxy)-thiocarbonylpiperidin und
(12) 4-[4-(Dimethylaminomethyl)benzyl]-N-(4-fluorphenoxy)-carbonylpiperidin,
deren Gemische und deren Salze.

6. Physiologisch verträgliche Salze der Verbindungen nach mindestens einem der Ansprüche 1 bis 5 mit anorganischen oder organischen Säuren.

7. Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Arzneimittel gemäß Anspruch 7, geeignet zur Behandlung von Krankheiten, bei denen die Cholesterol-Biosynthese eine Rolle spielt.

9. Arzneimittel gemäß Anspruch 8, enthaltend eine Verbindung nach mindestens einem der Ansprüche 1 bis 5 oder ein physiologisch verträgliches Salz gemäß Anspruch 6 in Kombination mit einem oder mehreren weiteren Wirkstoffen mit cholesterol- oder lipidsenkender Aktivität.

10. Arzneimittel gemäß Anspruch 9, **dadurch gekennzeichnet, daß** die weiteren Wirkstoffe ausgewählt sind aus
gallensäurebindenden Harzen,
Verbindungen, die die Cholesterolresorption hemmen,
Verbindungen, die über einen anderen Mechanismus als Hemmung der 2,3-Epoxisqualen-Lanosterol-Cyclase in die Cholesterolbiosynthese eingreifen,
Fibraten, Nikotinsäure, deren Derivate und Analoge, sowie Probucol.

11. Arzneimittel gemäß Anspruch 8, 9 oder 10, geeignet zur Behandlung oder Prophylaxe der Hypercholesterolämie, der Hyperlipoproteinämie, der Hypertriglyceridämie und den daraus resultierenden atherosklerotischen Gefäßveränderungen mit ihren Folgeerkrankungen wie koronare Herzkrankheit, cerebrale Ischämie, Claudicatio intermittens oder Gangrän, zur Behandlung von Erkrankungen, die mit überhöhter Zellproliferation im Zusammenhang stehen, zur Prophylaxe und Behandlung von Gallensteinleiden oder zur Behandlung von Mykosen.

12. Verwendung einer Verbindung nach mindestens einem der Ansprüche 1 bis 5 zur Herstellung eines Arzneimittels gemäß einem der Ansprüche 8 bis 11.

13. Futtermittel für Legehennen, enthaltend eine Verbindung nach mindestens einem der Ansprüche 1 bis 5 oder ein physiologisch verträgliches Salz gemäß Anspruch 6.

14. Verwendung einer Verbindung nach mindestens einem der Ansprüche 1 bis 6 zur Herstellung eines Futtermittels für Legehennen zur Erzeugung cholesterolarmer Eier.

15. Verfahren zur Herstellung eines Arzneimittels gemäß Anspruch 7, **dadurch gekennzeichnet, daß** auf nichtchemischem Wege eine Verbindung nach mindestens einem der Ansprüche 1 bis 6 in einen oder mehrere inerte Trägerstoffe und/oder Verdünnungsmittel eingearbeitet wird.

16. Verfahren zur Herstellung der Verbindungen nach mindestens einem der Ansprüche 1 bis 6, **dadurch gekennzeichnet, daß**
a) eine Verbindung der allgemeinen Formel 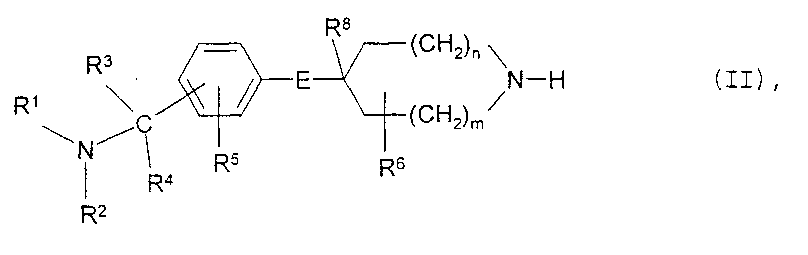 in der
m, n, E mit Ausnahme der Sulfinylgruppe, R¹ bis R⁶ und R⁸ wie in den Ansprüchen 1 bis 5 definiert sind, mit einer Verbindung der allgemeinen Formel 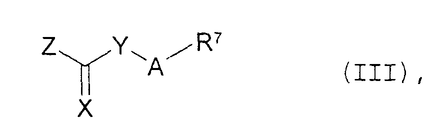 in der
A, X, Y und R⁷ wie in den Ansprüchen 1 bis 5 definiert sind und
Z eine Austrittsgruppe bedeutet, umgesetzt wird oder
b) zur Herstellung von Verbindungen der allgemeinen Formel (I), in der X und Y jeweils ein Schwefelatom bedeuten und m, n, A, E und R¹ bis R⁸ mit der Maßgabe wie in den Ansprüchen 1 bis 5 definiert sind, daß R⁷ keine gegebenenfalls substituierte Phenyl- oder Naphtylgruppe darstellt, falls A eine Einfachbindung bedeutet, eine Verbindung der allgemeinen Formel (II), in der m ,n ,E mit Ausnahme der Sulfinylgruppe, R¹ bis R⁶ und R⁸ wie in den Ansprüchen 1 bis 5 definiert sind, mit Schwefelkohlenstoff und anschließend mit einem Alkylierungsmittel der allgemeinen Formel 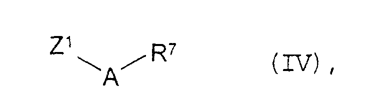 in der
A und R⁷ mit der Maßgabe wie in den Ansprüchen 1 bis 5 definiert sind, daß R⁷ keine gegebenenfalls substituierte Phenyloder Naphtylgruppe darstellt, falls A eine Einfachbindung bedeutet, und Z¹ eine Austrittsgruppe bedeutet, umgesetzt wird oder
c) zur Herstellung von Verbindungen der allgemeinen Formel (I), in der E eine Sulfinylgruppe bedeutet, eine Verbindung der allgemeinen Formel (I), in der m, n, A, X, Y, und R¹ bis R⁸ wie in den Ansprüchen 1 bis 5 definiert sind und E ein Schwefelatom bedeutet, oxidiert wird und
gewünschtenfalls ein so erhaltenes Gemisch der geometrischen Isomeren einer Verbindung der allgemeinen Formel I in ihre Enantiomeren und Diastereomeren aufgetrennt wird oder
eine so erhaltene Verbindung der allgemeinen Formel I in ihr Salz mit einer anorganischen oder organischen Säure, insbesondere in ihre physiologisch verträglichen Salze, übergeführt wird.

## Claims

1. Urethanes derived from azacycloalkanes and the thio and dithio analogues thereof of the general formula 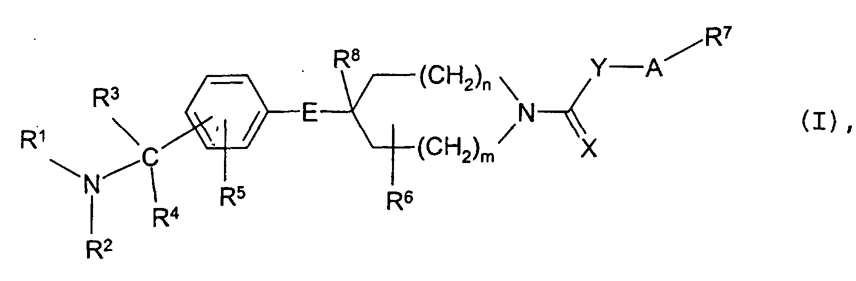 wherein
m denotes the numbers 0 or 1,
n denotes the numbers 1 or 2,
A denotes a single bond, a straight-chained or branched C₁₋₈-alkylene group, a C₂₋₈-alkenylene or C₂₋₈-alkynylene group, whilst an unsaturated group is not directly bound to the group Y,
X denotes an oxygen or sulphur atom,
Y denotes an oxygen or sulphur atom,
R¹ denotes a straight-chained or branched C₁₋₆-alkyl group, a C₁₋₄-alkenyl group or a C₁₋₄-alkynyl group, whilst the multiple bond is isolated from the nitrogen-carbon bond,
R² denotes a straight-chained or branched C₁₋₆-alkyl group, a C₁₋₄-alkenyl group or a C₁₋₄-alkynyl group, whilst the multiple bond is isolated from the nitrogen-carbon bond, or
R¹ and R² together with the nitrogen atom denotes a 5- to 7-membered, saturated heterocyclic ring wherein a methylene group isolated from the nitrogen atom may be replaced by an oxygen or sulphur atom or by an -NH- or -N(alkyl)- group,
R³ to R⁶, which may be identical or different, denote hydrogen atoms or alkyl groups,
R⁷ denotes a C₃₋₇-cycloalkyl group, a phenyl or naphthyl group optionally substituted by one or two halogen atoms or by an alkyl, alkoxy, trifluoromethyl or cyano group or, if A does not denote a single bond, R⁷ also denotes a hydrogen atom,
E denotes an oxygen or sulphur atom, a methylene, carbonyl or sulphinyl group and
R⁸ denotes a hydrogen atom or
E denotes the group -C(R⁹R¹⁰)-, wherein
R⁹ denotes a hydrogen atom and
R¹⁰ together with the adjacent group R⁸ denotes a carbon-carbon bond,
whilst, unless otherwise stated, alkyl groups contained in the groups mentioned above may contain 1 to 3 carbon atoms and a halogen atom mentioned above may be a fluorine, chlorine or bromine atom,
the enantiomers, diastereomers, the mixtures thereof and the salts thereof.

2. Compounds of general formula I according to claim 1, wherein
m denotes the number 1,
n denotes the number 1,
A denotes a single bond, a straight-chained or branched C₁₋₄-alkylene group or a C₂₋₄-alkenylene group, wherein an unsaturated group is not directly bound to the group Y,
X denotes an oxygen or sulphur atom,
Y denotes an oxygen or sulphur atom,
R¹ denotes a straight-chained or branched C₁₋₆-alkyl group, an allyl or propargyl group, whilst the multiple bond is isolated from the nitrogen-carbon bond,
R² denotes a straight-chained or branched C₁₋₆-alkyl group, an allyl or propargyl group, whilst the multiple bond is isolated from the nitrogen-carbon bond,
R¹ and R² together with the nitrogen atom denote a 5- to 7-membered, saturated heterocyclic ring wherein a methylene group isolated from the nitrogen atom may be replaced by an oxygen or sulphur atom,
R³ to R⁶, which may be identical or different, denote hydrogen atoms or methyl groups,
R⁷ denotes a C₃₋₆-cycloalkyl group, a phenyl or naphthyl group optionally substituted by one or two halogen atoms or by an alkyl, alkoxy, trifluoromethyl or cyano group,
E denotes a sulphur atom, a methylene, carbonyl or sulphinyl group and
R⁸ denotes a hydrogen atom or
E denotes the group -C(R⁹R¹⁰)- whilst
R⁹ denotes a hydrogen atom and
R¹⁰ together with the adjacent group R⁸ denotes a carbon-carbon bond,
whilst, unless otherwise stated, alkyl groups contained in the groups mentioned above may each contain 1 to 3 carbon atoms and a halogen atom mentioned above may be a fluorine, chlorine or bromine atom,
the enantiomers, diastereomers, the mixtures thereof and the salts thereof.

3. Compounds of general formula I according to claim 1 wherein
m denotes the number 1,
n denotes the number 1,
A denotes a single bond or a straight-chained or branched C₁₋₃ alkylene group,
X denotes an oxygen or sulphur atom,
Y denotes an oxygen or sulphur atom,
R¹ denotes a straight-chained or branched C₁₋₃-alkyl group,
R² denotes a straight-chained or branched C₁₋₃-alkyl group, or
R¹ and R² together with the nitrogen atom denote a piperidino or morpholino group,
R³ to R⁶ denote hydrogen atoms,
R⁷ denotes a cyclohexyl group or a phenyl group optionally substituted by,a halogen atom or by an alkyl, alkoxy or trifluoromethyl group,
E denotes a sulphur atom, a methylene group, a carbonyl or sulphinyl group and
R⁸ denotes a hydrogen atom or
E denotes the group -C(R⁹R¹⁰)-, wherein
R⁹ denotes a hydrogen atom and
R¹⁰ together with the adjacent group R⁸ denotes a carbon-carbon bond,
the enantiomers, diastereomers, the mixtures thereof and the salts thereof.

4. Compounds of general formula I according to claim 1 wherein
m denotes the number 1,
n denotes the number 1,
A denotes a single bond or a methylene group,
X denotes an oxygen or sulphur atom,
Y denotes an oxygen or sulphur atom,
R¹ and R² each denote a methyl group,
R³ to R⁶ denote hydrogen atoms,
R⁷ denotes a phenyl group optionally substituted by a fluorine or chlorine atom or by a methyl group,
E denotes a sulphur atom, a methylene or carbonyl group and
R⁸ denotes a hydrogen atom or
E denotes the group -C(R⁹R¹⁰)-, wherein
R⁹ denotes a hydrogen atom and
R¹⁰ together with the adjacent group R⁸ denotes a carbon-carbon bond,
the mixtures and salts thereof.

5. The following compounds of general formula I according to claim 1:
(1) N-(benzylthio)thiocarbonyl-4-[4-(dimethylaminomethyl) -phenylthio]piperidine,
(2) N-(benzylthio)thiocarbonyl-4-[4-(dimethylaminomethyl)ben-zoyl]piperidine,
(3) N-benzyloxycarbonyl-4-[4-(dimethylaminomethyl)phenylthio]-piperidine,
(4) N-(4-chlorophenoxy)carbonyl-4-[4-(dimethylaminomethyl)phenylthio]piperidine,
(5) N-(benzylthio)thiocarbonyl-4-[4-(dimethylaminomethyl)benzyl]piperidine,
(6) N-(benzylthio)thiocarbonyl-4-[4-(dimethylaminomethyl)benzylidene]piperidine,
(7) N-(4-chlorophenoxy)thiocarbonyl-4-[4-(dimethylaminomethyl)-benzyl]piperidine,
(8) N-(4-chlorophenoxy)carbonyl-4-[4-(dimethylaminomethyl)-benzyl]piperidine,
(9) N-benzyloxycarbonyl-4-[4-(dimethylaminomethyl]benzyl]-piperidine,
(10) 4-[4-(dimethylaminomethyl)benzyl]-N- (4-methylphenoxy) -carbonylpiperidine,
(11) 4-[4-(dimethylaminomethyl)benzyl]-N-(4-methylphenoxy)-thiocarbonylpiperidine and
(12) 4-[4-(dimethylaminomethyl)benzyl]-N-(4-fluorphenoxy)-carbonylpiperidine,
the mixtures and the salts thereof.

6. Physiologically acceptable salts of the compounds according to at least one of claims 1 to 5 with inorganic or organic acids.

7. Pharmaceutical compositions containing a compound according to at least one of claims 1 to 5 or a physiologically acceptable salt according to claim 6 optionally together with one or more inert carriers and/or diluents.

8. Pharmaceutical compositions according to claim 7, suitable for treating diseases in which cholesterol biosynthesis plays a part.

9. Pharmaceutical compositions according to claim 8, containing a compound according to at least one of claims 1 to 5 or a physiologically acceptable salt according to claim 6 in combination with one or more other active substances with a cholesterol- or lipid-lowering activity.

10. Pharmaceutical compositions according to claim 9, **characterised in that** the other active substances are selected from
resins which bind bile acid,
compounds which inhibit cholesterol absorption,
compounds which are involved in cholesterol biosynthesis by a mechanism other than the inhibition of 2,3-epoxysqualene-lanosterol-cyclase,
fibrates, nicotinic acid, the derivatives and analogues thereof, and probucol.

11. Pharmaceutical compositions according to claim 8, 9 or 10, suitable for the treatment or prophylaxis of hypercholesterolaemia, hyperlipoproteinaemia, hypertriglyceridaemia and the resulting atherosclerotic vascular changes with their consequent diseases such as coronary heart disease, cerebral ischaemia, Claudicatio intermittens or gangrene, for treating diseases connected with excessive cell proliferation, for the prophylaxis and treatment of gallstone problems or for treating mycoses.

12. Use of a compound according to at least one of claims 1 to 5 for preparing a pharmaceutical composition according to one of claims 8 to 11.

13. Feed for laying hens, containing a compound according to at least one of claims 1 to 5 or a physiologically acceptable salt according to claim 6.

14. Use of a compound according to at least one of claims 1 to 6 for preparing a feed for laying hens for producing low-cholesterol eggs.

15. Process for preparing a pharmaceutical composition according to claim 7, **characterised in that** a compound according to at least one of claims 1 to 6 is incorporated in one or more inert carriers and/or diluents by a non-chemical method.

16. Process for preparing the compounds according to at least one of claims 1 to 6, **characterised in that**
a) a compound of general formula 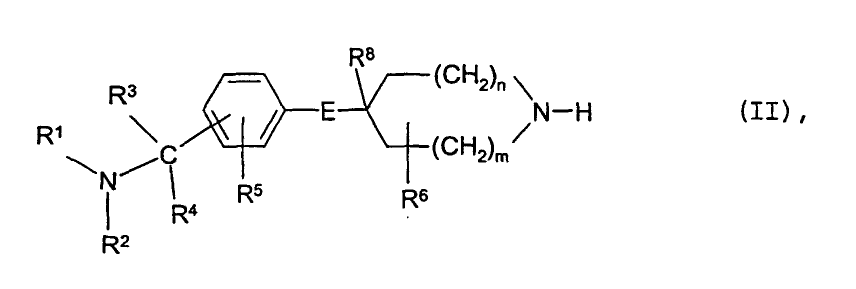 wherein
m, n, E with the exception of the sulphinyl group, R¹ to R⁶ and R⁸ are defined as in claims 1 to 5, is reacted with a compound of general formula 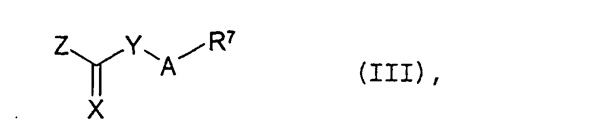 wherein
A, X, Y and R⁷ are defined as in claims 1 to 5 and Z denotes a leaving group, or
b) in order to prepare compounds of general formula (I), wherein X and Y each denote a sulphur atom and m, n, A, E and R¹ to R⁸ are defined as in claims 1 to 5 with the proviso that R⁷ does not represent an optionally substituted phenyl or naphthyl group if A denotes a single bond:
a compound of general formula (II), wherein m, n and E with the exception of the sulphinyl group, R¹ to R⁶ and R⁸ are defined as in claims 1 to 5, is reacted with carbon disulphide and subsequently with an alkylating agent of general formula 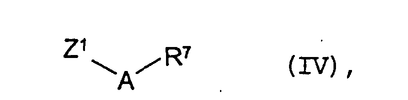 wherein
A and R⁷ are defined as in claims 1 to 5, with the proviso that R⁷ does not represent an optionally substituted phenyl or naphthyl group if A denotes a single bond, and Z¹ denotes a leaving group, or
c) in order to prepare compounds of general formula (I) wherein E denotes a sulphinyl group:
a compound of general formula (I) wherein m, n, A, X, Y, and R¹ to R⁸ are defined as in claims 1 to 5 and E denotes a sulphur atom, is oxidised, and
if desired a mixture of the geometric isomers of a compound of general formula I thus obtained is resolved into its enantiomers and diastereomers or
a compound of general formula I thus obtained is converted into a salt thereof with an inorganic or organic acid, particularly into the physiologically acceptable salts thereof.

## Revendications

1. Uréthanes dérivés d'azacycloalcanes et leurs analogues thio et dithio de formule générale 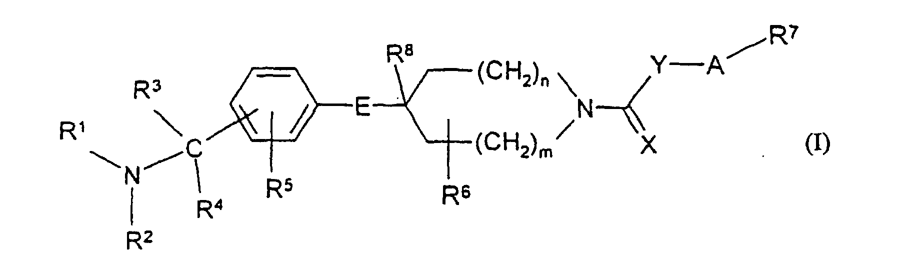 dans laquelle
m représente le chiffre 0 ou 1,
n représente le chiffre 1 ou 2,
A représente une liaison simple, un groupe alkylène en C₁-C₈ linéaire ou ramifié, un groupe alcénylène en C₂-C₈ ou alcynylène en C₂-C₈, dans lequel un groupe insaturé n'est pas lié directement au reste Y,
X représente un atome d'oxygène ou de soufre,
Y représente un atome d'oxygène ou de soufre,
R¹ représente un groupe alkyle en C₁-C₆ linéaire ou ramifié, un groupe alcényle en C₁-C₄ ou un groupe alcynyle en C₁-C₄, dans lequel la liaison multiple est isolée de la liaison azote-carbone,
R² représente un groupe alkyle en C₁-C₆ linéaire ou ramifié, un groupe alcényle en C₁-C₄ ou alcynyle en C₁-C₄, la liaison multiple étant isolée de la liaison azote-carbone, ou bien
R¹ et R² forment ensemble avec l'atome d'azote un hétérocycle saturé de 5 à 7 chaînons dans lequel un groupe méthylène isolé de l'atome d'azote peut être remplacé par un atome d'oxygène ou de soufre ou par un groupe -NH- ou -N(alkyle)-,
R³ à R⁶, qui peuvent être identiques ou différents, représentent des atomes d'hydrogène ou des groupes alkyle,
R⁷ représente un groupe cycloalkyle en C₃-C₇, un groupe phényle ou naphtyle éventuellement substitué par un ou deux atomes d'halogène ou par un groupe alkyle, alcoxy, trifluorométhyle ou cyano ou, dans la mesure où A ne représente pas une liaison simple, R⁷ peut aussi représenter un atome d'hydrogène,
E représente un atome d'oxygène ou de soufre ou un groupe méthylène, carbonyle ou sulfinyle, et
R⁸ est un atome d'hydrogène, ou
E représente le groupe -C(R⁹R¹⁰)- dans lequel
R⁹ représente un atome d'hydrogène et
R¹⁰ représente avec le groupe R⁸ voisin une liaison carbone-carbone,
et où, sauf indication contraire, les groupes alkyle contenus dans les restes précités peuvent contenir chacun de 1 à 3 atomes de carbone, et un atome d'halogène précité peut représenter un atome de fluor, de chlore ou de brome,
leurs énantiomères, leurs diastéréoisomères, leurs mélanges et leurs sels.

2. Composés de formule générale I selon la revendication 1, dans laquelle
m représente le chiffre 1,
n représente le chiffre 1,
A représente une liaison simple, un groupe alkylène en C₁-C₄ linéaire ou ramifié ou un groupe alcénylène en C₂-C₄, dans lequel un groupe insaturé n'est pas lié directement au reste Y,
X représente un atome d'oxygène ou de soufre,
Y représente un atome d'oxygène ou de soufre,
R¹ représente un groupe alkyle en C₁-C₆ linéaire ou ramifié ou un groupe allyle ou propargyle, dans lequel la liaison multiple est isolée de la liaison azote-carbone,
R² représente un groupe alkyle en C₁-C₆ linéaire ou ramifié ou un groupe allyle ou propargyle, dans lequel la liaison multiple est isolée de la liaison azote-carbone, ou bien
R¹ et R² forment ensemble avec l'atome d'azote un cycle hétérocyclique saturé de 5 à 7 chaînons dans lequel un groupe méthylène isolé de l'atome d'azote peut être remplacé par un atome d'oxygène ou de soufre,
R³ à R⁶, qui peuvent être identiques ou différents, représentent des atomes d'hydrogène ou des groupes méthyle,
R⁷ représente un groupe cycloalkyle en C₃-C₆ ou un groupe phényle ou naphtyle éventuellement substitué par un ou deux atomes d'halogène ou par un groupe alkyle, alcoxy, trifluorométhyle ou cyano,
E représente un atome de soufre ou un groupe méthylène, carbonyle ou sulfinyle, et
R⁸ est un atome d'hydrogène, ou bien
E représente le groupe -C(R⁹R¹⁰)- dans lequel
R⁹ représente un atome d'hydrogène et
R¹⁰ représente avec le groupe R⁸ voisin une liaison carbone-carbone,
et où, sauf indication contraire, les groupes alkyle contenus dans les restes précités peuvent contenir chacun de 1 à 3 atomes de carbone, et un atome d'halogène précité peut représenter un atome de fluor, de chlore ou de brome,
leurs énantiomères, leurs diastéréoisomères, leurs mélanges et leurs sels.

3. Composés de formule générale I selon la revendication 1, dans laquelle
m représente le chiffre 1,
n représente le chiffre 1,
A représente une liaison simple ou un groupe alkylène en C₁-C₃ linéaire ou ramifié,
X représente un atome d'oxygène ou de soufre,
Y représente un atome d'oxygène ou de soufre,
R¹ représente un groupe alkyle en C₁-C₃ linéaire ou ramifié,
R² représente un groupe alkyle en C₁-C₃ linéaire ou ramifié, ou bien
R¹ et R² forment ensemble avec l'atome d'azote un groupe pipéridino ou morpholino,
R³ à R⁶ représentent des atomes d'hydrogène,
R⁷ représente un groupe cyclohexyle ou un groupe phényle éventuellement substitué par un atome d'halogène ou par un groupe alkyle, alcoxy ou trifluorométhyle,
E représente un atome de soufre ou un groupe méthylène, carbonyle ou sulfinyle, et
R⁸ est un atome d'hydrogène, ou bien
E représente le groupe -C(R⁹R¹⁰)- dans lequel
R⁹ représente un atome d'hydrogène et
R¹⁰ représente avec le groupe R⁸ voisin une liaison carbone-carbone,
leurs énantiomères, leurs diastéréoisomères, leurs mélanges et leurs sels.

4. Composés de formule générale I selon la revendication 1, dans laquelle
m représente le chiffre 1,
n représente le chiffre 1,
A représente une liaison simple ou un groupe méthylène,
X représente un atome d'oxygène ou de soufre,
Y représente un atome d'oxygène ou de soufre,
R¹ et R² représentent chacun un groupe méthyle,
R³ à R⁶ représentent des atomes d'hydrogène,
R⁷ représente un groupe phényle éventuellement substitué par un atome de fluor ou de chlore ou par un groupe méthyle,
E représente un atome de soufre ou un groupe méthylène ou carbonyle, et
R⁸ est un atome d'hydrogène, ou bien
E représente le groupe -C(R⁹R¹⁰)- dans lequel
R⁹ représente un atome d'hydrogène et
R¹⁰ représente avec le groupe R⁸ voisin une liaison carbone-carbone,
leurs mélanges et leurs sels.

5. Composés de formule générale I selon la revendication 1, suivants :
(1) N-(benzylthio)thiocarbonyl-4-[4-(diméthylaminométhyl)phénylthio]pipéridine,
(2) N-(benzylthio)thiocarbonyl-4-[4-(diméthylaminométhyl)benzoyl]pipéridine,
(3) N-benzyloxycarbonyl-4-[4-(diméthylaminométhyl)phénylthio]pipéridine,
(4) N-(4-chlorophénoxy)carbonyl-4-[4-(diméthylaminométhyl)phénylthio]pipéridine,
(5) N-(benzylthio)thiocarbonyl-4-[4-(diméthylaminométhyl)benzyl]pipéridine,
(6) N-(benzylthio)thiocarbonyl-4-[4-(diméthylaminométhyl)benzylidène]pipéridine,
(7) N-(4-chlorophénoxy)thiocarbonyl-4-[4-(diméthylaminométhyl)benzyl]pipéridine,
(8) N-(4-chlorophénoxy)carbonyl-4-[4-(diméthylaminométhyl)benzyl]pipéridine,
(9) N-benzyloxycarbonyl-4-[4-(diméthylaminométhyl)benzyl]pipéridine,
(10) 4-[4-(diméthylaminométhyl)benzyl]-N-(4-méthylphénoxy)carbonylpipéridine,
(11) 4-[4-(diméthylaminométhyl)benzyl]-N-(4-méthylphénoxy)thiocarbonylpipéridine et
(12) 4-[4-(diméthylaminométhyl)benzyl]-N-(4-fluorophénoxy)carbonylpipéridine,
leurs mélanges et leurs sels.

6. Sels acceptables d'un point de vue physiologique des composés selon l'une au moins des revendications 1 à 5 avec des acides inorganiques ou organiques.

7. Médicament contenant un composé selon l'une au moins des revendications 1 à 5 ou un sel acceptable d'un point de vue physiologique selon la revendication 6, avec éventuellement un ou plusieurs supports et/ou diluants inertes.

8. Médicament selon la revendication 7, approprié au traitement de maladies dans lesquelles intervient la biosynthèse du cholestérol.

9. Médicament selon la revendication 8, contenant un composé selon l'une au moins des revendications 1 à 5 ou un sel acceptable d'un point de vue physiologique selon la revendication 6, en combinaison avec une ou plusieurs autres substances actives ayant une activité hypocholestérolémiante ou hypolipémiante.

10. Médicament selon la revendication 9, **caractérisé en ce que** les autres substances actives sont choisies parmi
des résines se liant aux acides biliaires,
des composés inhibant la résorption du cholestérol,
des composés qui interviennent dans la biosynthèse du cholestérol par un autre mécanisme que l'inhibition de la 2,3-époxysqualène-lanostérol-cyclase,
des fibrates, l'acide nicotinique, leurs dérivés et analogues, et le probucol.

11. Médicament selon la revendication 8, 9 ou 10, approprié au traitement ou à la prophylaxie de l'hypercholestérolémie, de l'hyperlipoprotéinémie, de l'hypertriglycéridémie et des altérations vasculaires athéroscléreuses qui en résultent, avec leurs complications comme l'insuffisance coronarienne, l'ischémie cérébrale, la claudication intermittente ou la gangrène, au traitement de maladies qui sont en relation avec une prolifération excessive des cellules, à la prophylaxie et au traitement de calculs biliaires ou au traitement de mycoses.

12. Utilisation d'un composé selon l'une au moins des revendications 1 à 5 pour la préparation d'un médicament selon l'une des revendications 8 à 11.

13. Aliment pour poules pondeuses, contenant un composé selon l'une au moins des revendications 1 à 5 ou un sel physiologique acceptable selon la revendication 6.

14. Utilisation d'un composé selon l'une au moins des revendications 1 à 5 pour la préparation d'un aliment pour poules pondeuses destiné à l'obtention d'oeufs à faible teneur en cholestérol.

15. Procédé de préparation d'un médicament selon la revendication 7, **caractérisé en ce que** l'on incorpore par des voies non chimiques un composé selon l'une au moins des revendications 1 à 6 dans un ou plusieurs supports et/ou diluants inertes.

16. Procédé de préparation des composés selon l'une au moins des revendications 1 à 6, **caractérisé en ce que**
a) on fait réagir un composé de formule générale 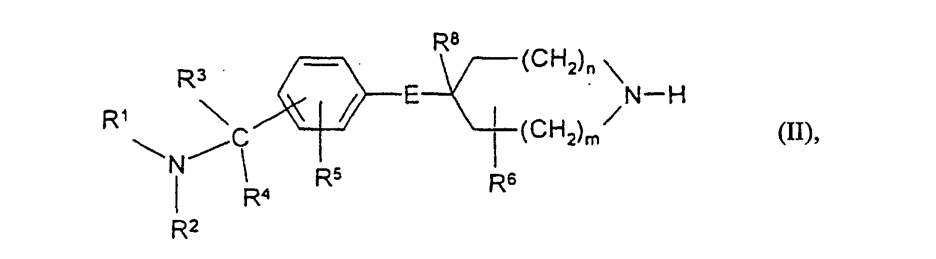 dans laquelle m, n, E à l'exception du groupe sulfinyle, R¹ à R⁶ et R⁸ sont tels que définis dans les revendications 1 à 5, avec un composé de formule générale 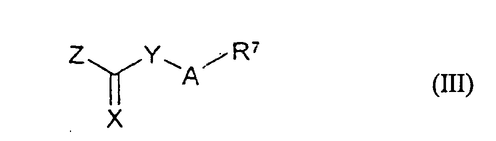 dans laquelle
A, X, Y et R⁷ sont tels que définis dans les revendications 1 à 5, et
Z représente un groupe partant, ou
b) pour préparer des composés de formule générale (I) dans laquelle X et Y représentent chacun un atome de soufre et m, n, A, E et R¹ à R⁸ sont tels que définis dans les revendications 1 à 5 à condition que R⁷ ne représente pas un groupe phényle ou naphtyle éventuellement substitué dans le cas où A représente une liaison simple, on fait réagir un composé de formule générale (II) dans laquelle m, n, E à l'exception du groupe sulfinyle, R¹ à R⁶ et R⁸ sont tels que définis dans les revendications 1 à 5, avec du sulfure de carbone, puis avec un agent d'alkylation de formule générale 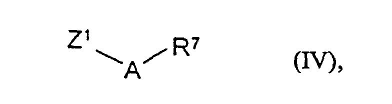 dans laquelle A et R⁷ sont tels que définis dans les revendications 1 à 5, à condition que R⁷ ne représente pas un groupe phényle ou naphtyle éventuellement substitué dans le cas où A représente une liaison simple, et Z¹ représente un groupe partant, ou
c) pour préparer des composés de formule générale (I) dans laquelle E représente un groupe sulfinyle, on oxyde un composé de formule générale (I) dans laquelle m, n, A, X, Y et R¹ à R⁸ sont tels que définis dans les revendications 1 à 5 et E représente un atome de soufre, et
si désiré, on sépare un mélange ainsi obtenu des isomères géométriques d'un composé de formule générale I en ses énantiomères et diastéréoisomères, ou
on transforme un composé de formule générale I ainsi obtenu en son sel avec un acide inorganique ou organique, en particulier en ses sels acceptables d'un point de vue physiologique.
